# EUROPEAN PATENT APPLICATION

(11) **EP 1 559 707 A1**
(43) Date of publication of application: **03.08.2005**
(21) Application number: 03717627.8
(22) Date of filing: 17.04.2003
(51) Int. Cl.: C07C 217/18, A61K 31/138, A61K 31/40, A61K 31/4453, A61K 31/495, A61P 15/12, A61P 19/10, A61P 35/00, A61P 43/00, C07D 211/22, C07D 295/088, A61K 31/55, A61K 31/445, A61K 31/451, C07D 295/096, C07D 211/14

(54) **SPIRO COMPOUNDS, MEDICINAL COMPOSITIONS CONTAINING THE SAME AND INTERMEDIATES OF THE COMPOUNDS**

(30) Priority: 01.10.2002 JP 2002289187
(71) Applicant: Dainippon Pharmaceutical Co., Ltd., Osaka-shi, Osaka 541-8524 (JP)
(72) Inventor: WATANABE, Nobuhide, Toyonaka-shi, Osaka 560-0085 (JP); NAKAGAWA, Hiroshi, Suita-shi, Osaka 564-0062 (JP); TSUJI, Jun-ichi, Otsu-shi, Shiga 520-0246 (JP); MINATO, Hisao, Sanda-shi, Hyogo 669-1547 (JP); IKENO, Akihisa, Suita-shi, Osaka 565-0824 (JP); KOHAYAKAWA, Chie, Toyonaka-shi, Osaka 561-0853 (JP)
(74) Representative: von Kreisler, Alek, Dipl.-Chem.
(86) International application number: PCT/JP2003/004889
(87) International publication number: WO 2004/031125

(57) **Abstract**

A spiro compound represented by the following formula (I) 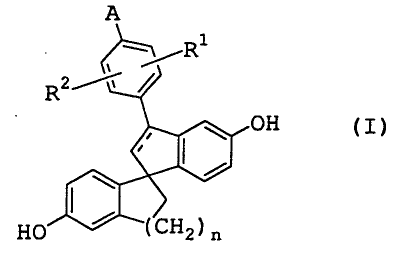 wherein R¹ and R² are the same or different and each is a hydrogen atom, a chlorine atom and the like, n is 1, 2 or 3, a bond containing a broken line is a single bond or a double bond, A is -X-(CH₂)_{q}-N(R³)(R⁴); a group represented by the following formula (a) 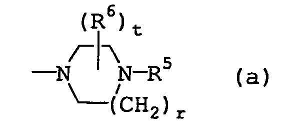 and the like, wherein X is an oxygen atom or a sulfur atom, q is 2 or 3, R³ and R⁴ are the same or different and each is a C₁₋₆ alkyl group and the like, or R³ and R⁴ optionally form, together with the adjacent nitrogen atom, a piperidine ring and the like optionally substituted by one or two C₁₋₆ alkyl and the like, R⁵ is a C₁₋₆ alkyl group and the like, R⁶ is a hydrogen atom and the like, and r and t are each independently one or two, or a pharmaceutically acceptable acid addition salt thereof. The compound is useful as a selective estrogen receptor modulator having a climacteric syndrome-ameliorating effect, and can be expected to be a drug for the prophylaxis and/or treatment of osteoporosis, climacteric syndrome and breast cancer.

## Description

### Technical Field

The present invention relates to novel spiro compounds that act as a selective estrogen receptor modulator. More particularly, this invention relates to a spiro[indene-1,1'-indan] derivatives and compounds related thereto, pharmaceutical compositions comprising the same and intermediates for the compounds.

### BACKGROUND ART

Female ovarian function rapidly decreases in several years before and after menopause, and postmenopausal blood estrogen level becomes not more than 1/10 of that before menopause. Therefore, postmenopausal females tend to express, besides what is called a climacteric syndrome, conditions due to the shortage of estrogen, such as lipidosis, arteriosclerotic disease, osteoporosis, dysmnesia, cognitive impairment and the like. To ameliorate such various conditions of postmenopausal females, hormone replacement therapy using estrogen and progestin in combination (hereinafter to be referred to as HRT) or estrogen replacement therapy (hereinafter to be referred to as ERT) has been applied.

Hot flash, which is the main complaint of the climacteric syndrome, is remarkably ameliorated by HRT and ERT. It has been established that severity and frequency of osteoporosis can be reduced by these replacement therapies. Moreover, it has been reported that these replacement therapies show desirable effects on lipid metabolism, cardiovascular system and psychoneurotic system. However, HRT and ERT are associated with problems in that the incidence of breast cancer and endometrioma increases, side effects such as mammalgia, genital bleeding and the like are expressed at high frequency and the like.

To reduce such side effects, therefore, the development of a pharmaceutical agent having only the desirable objective activities from various estrogenic actions expressed by HRT and ERT is proceeding. At present, raloxifene hydrochloride ([6-hydroxy-2-(4-hydroxyphenyl)benzo[b]thien-3-yl][4-[2-(1-piperidinyl)ethoxy]phenyl]-methanone hydrochloride) has been clinically used as a therapeutic agent for osteoporosis and tamoxifen citrate ((Z)-2-[4-(1,2-diphenyl-1-butenyl)phenoxy]-N,N-dimethylethanamine citrate) has been clinically used as an agent for the prophylaxis or treatment of breast cancer. These pharmaceutical agents are called selective estrogen receptor modulators (hereinafter to be referred to as SERM), because they show an estrogenic activity and an antiestrogenic activity at various ratios and levels for respective target organs. While raloxifene hydrochloride acts as an estrogen agonist for the bone and lipid metabolisms, it acts as an estrogen antagonist for the genital organs such as uterus, breast and the like. On the other hand, tamoxifen citrate also acts as an estrogen agonist for the bone and lipid metabolisms, like raloxifene hydrochloride, and acts as an estrogen antagonist for breast. However, it acts as a partial agonist for uterus, unlike raloxifene hydrochloride. Such difference in the activity characteristics depends on the chemical structure of each pharmaceutical agent, which is considered to be based on the difference in the steric structure of the compound-receptor complex. This means that a novel SERM having different activity characteristics can be created.

An ideal SERM acts as an estrogen agonist for the tissues expecting an estrogenic activity and simultaneously acts as an estrogen antagonist for the tissues for which an estrogenic activity is not preferable. Use of such SERM makes it possible to alleviate deficiency symptom of estrogen, while preventing the development of breast cancer and endometrioma, or treating them. However, SERMs clinically used at present are not pharmaceutical agents fully satisfied as such an ideal SERM. To be precise, it is known that raloxifene hydrochloride and tamoxifen citrate reduce the incidence of breast cancer and show a prophylactic or therapeutic effect on postmenopausal osteoporosis as well as a lipid metabolism-improving action, but show no ameliorating action on hot flash, which is the main complaint of the climacteric syndrome, and rather, aggravate hot flash [Doren M, J. Endocrinol. Invest., 22, 625 (1999)].

WO98/02151 describes that a compound represented by the following formula is a chemokine receptor antagonist, and useful as a therapeutic drug for the diseases associated with aberrant leukocyte recruitment and/or activation. However, this reference does not contain a description relating to its usefulness as SERM: 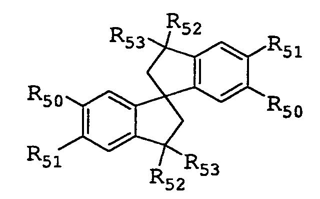 wherein R₅₀ and R₅₁ are each independently -OH, a halogen and the like,
R₅₂ and R₅₃ are each independently H, an aliphatic group, a substituted aliphatic group, a halogen, -NH₂, -NH (aliphatic group), -NH (substituted aliphatic group), -N (aliphatic group)₂ or -N (substituted aliphatic group)₂.

WO02/46134 describes that a 1,1',3,3'-tetrahydro-2,2'-spirobi(2H-indene) derivative represented by the following formula is an estrogen receptor ligand: 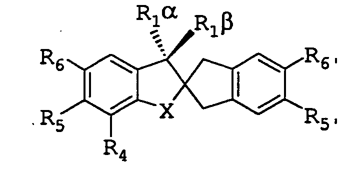 wherein R_{1α} and Rip are the same or different and each is a group selected from hydroxyl, R^{A} or OR^{A}, and the like, R^{A} is selected from hydrogen, alkyl, alkenyl, alkynyl, cycloalkyl, cycloalkylalkyl, aryl and arylalkyl, provided that R₁α and R₁β are not both H, R₁α is not OH when R₁β is H, and R₁β is not OH when R₁α is hydrogen, X is a methylene group (-CH₂-), an ethylene group (-CH₂CH₂-), or a substituted methylene group (-CR^{B}H-), wherein R^{B} is a C₁₋₄ alkyl group,
R₄ is a hydrogen atom and the like,
R₅, R₆, R₅, and R₆, are the same or different and each is a hydrogen atom, a hydroxyl group and the like.

The above-mentioned estrogen receptor ligand is a compound wherein a spiro bond is formed at the 2-positions of both indan and indan, or at the 2-positions of both indan and 1,2,3,4-tetrahydronaphthalene, which has a chemical structure different from that of the compound of the formula (I) to be mentioned later.

T.A. Blizzard et al. describe in Books of Abstracts 224th ACS National Meeting Boston, MA, August 18-22, 2002, DIVISION OF MEDICINAL CHEMISTRY, MEDI357 (US) that the following compound is SERM:

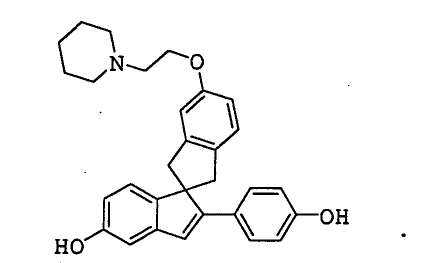

The above-mentioned compound has a different chemical structure from the compound of the formula (I) to be mentioned later, because it has a spiro bond at the 2-position of indan, and further has a 2-piperidinoethoxy group in the benzene ring of indan.

In addition, WO02/091993 encompassing the above-mentioned compound was published on November 21, 2002: 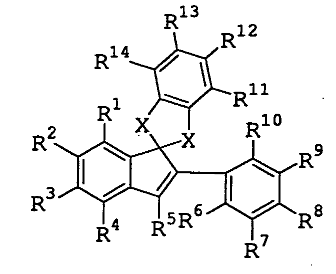 wherein each X is independently a group selected from CH₂, C=O, C=CH₂, C=NOR^{a}, CHCH₃, CHF, CHOH, C(CH₃)OH, CF₂ and S; R¹, R², R³, R⁴, R⁶, R⁷, R⁸, R⁹ and R¹⁰ are each independently a group selected from R^{a}, OR^{a} and the like; R¹¹, R¹², R¹³ and R¹⁴ are each independently a group selected from H, R^{b}, OR^{b} and the like;
R⁵ is a group selected from H, F and C₁₋₆ alkyl;
R^{a} is a group selected from H, C₁₋₆ alkyl and C₁₋₆ acyl;
R^{b} is a group selected from H, C₁₋₆ alkyl and C₁₋₆ acyl, wherein the alkyl and the acyl group are optionally substituted by an R^{c} group;
R^{c} is a group selected from OR^{d} and NR^{d}R^{e},
R^{d} and R^{e} are each independently and R⁵ a group selected from a group consisting of H and C₁₋₇ alkyl;
or R^{d} and R^{e} may form a 4- to 8-membered ring together with a nitrogen atom bonded thereto, wherein the ring may be interrupted by one O, NH, NCH₃ or S as well as may be substituted by one, two, three or four C₁₋₂ alkyl group(s) or one or two R^{f} group(s); and
R^{f} is a group selected from CH₂OH and CH₂CH₂OH.

### DISCLOSURE OF THE INVENTION

The present inventors conducted intensive studies in an attempt to provide a more highly useful SERM which prevents the incidence of breast cancer and endometrioma, or treats them, as well as enables prophylaxis or treatment of postmenopausal lipidosis and osteoporosis, by acting as an estrogen antagonist for the genital organs such as uterus, breast and the like, and acting as an estrogen agonist for lipid metabolism, bone, cardiovascular system and brain, and which is further expected to ameliorate the climacteric syndrome that the conventional SERMs have failed. As a result, they have found that the spiro compound represented by the formula (I) to be mentioned later meets the objects, which resulted in the completion of the present invention.

Accordingly, the present invention provides the following.
[1] A spiro compound represented by the following formula (I): 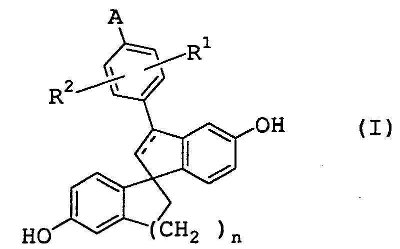 wherein R¹ and R² are the same or different and each is a hydrogen atom, a fluorine atom, a chlorine atom or a C₁₋₆ alkyl group,
   n is 1, 2 or 3,
   a bond containing a broken line is a single bond or a double bond,
   A is -(CH₂)ₚ-N(R³)(R⁴); -X-(CH₂)_{q}-N(R³)(R⁴); a group represented by the following formula (a): 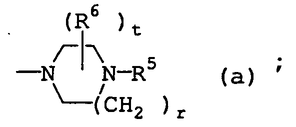 a group represented by the following formula (b) 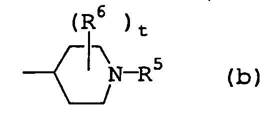 or a group represented by the following formula (c): 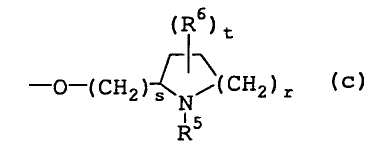 wherein p is 1, 2 or 3,
   X is an oxygen atom or a sulfur atom,
   q is 2 or 3,
   R³ and R⁴ are the same or different and each is a C₁₋₆ alkyl group or a phenyl C₁₋₄ alkyl group, or R³ and R⁴ optionally form, together with the adjacent nitrogen atom, a pyrrolidine ring, a piperidine ring, a homopiperidine ring, a piperazine ring or a morpholine ring, each optionally substituted by one or two groups selected from a C₁₋₆ alkyl, phenyl and benzyl,
   R⁵ is a C₁₋₆ alkyl group, a C₃₋₈ cycloalkyl group or a C₃₋₈ cycloalkyl C₁₋₄ alkyl group,
   R⁶ is a hydrogen atom or a C₁₋₄ alkyl group, and
   r, s and t are each independently one or two, or a pharmaceutically acceptable acid addition salt thereof.
[2] The compound of the above-mentioned [1], wherein A is -X-(CH₂)_{q}-N(R³)(R⁴) (wherein X, R³, R⁴ and q are as defined in the above-mentioned [1]), or a pharmaceutically acceptable acid addition salt thereof.
[3] A spiro compound represented by the following formula (I-1a): 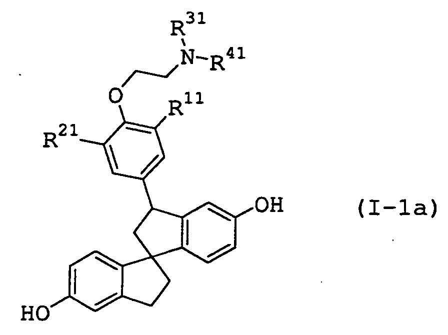 wherein R¹¹ and R²¹ are the same or different and each is a hydrogen atom, a fluorine atom or a chlorine atom,
   R³¹ and R⁴¹ form, together with the adjacent nitrogen atom, a pyrrolidine ring, a piperidine ring or a homopiperidine ring, each optionally substituted by one or two methyl groups, or a pharmaceutically acceptable acid addition salt thereof.
[4] A spiro compound represented by the following formula (I-2a) : 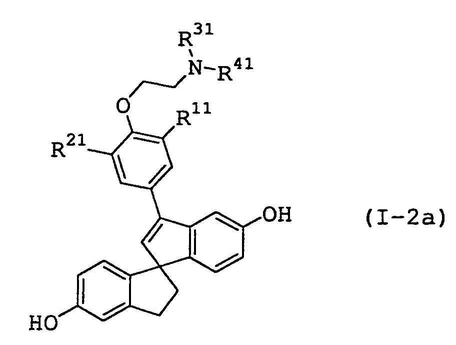 wherein R¹¹ and R²¹ are the same or different and each is a hydrogen atom, a fluorine atom or a chlorine atom,
   R³¹ and R⁴¹ form, together with the adjacent nitrogen atom, a pyrrolidine ring, a piperidine ring or a homopiperidine ring, each optionally substituted by one or two methyl groups, or a pharmaceutically acceptable acid addition salt thereof.
[5] (1R*,3S*)-3-[4-(2-Piperidinoethoxy)phenyl]-1,1'-spirobiindan-5,5'-diol, or a pharmaceutically acceptable acid addition salt thereof.
[6] 3-[4-(2-Piperidinoethoxy)phenyl]spiro[indene-1,1'-indan]-5,5'-diol, or a pharmaceutically acceptable acid addition salt thereof.
[7] A compound selected from (+)-3-[4-(2-piperidinoethoxy)phenyl]spiro[indene-1,1'-indan]-5,5'-diol and (-)-3-[4-(2-piperidinoethoxy)phenyl]spiro[indene-1,1'-indan]-5,5'-diol, or a pharmaceutically acceptable acid addition salt thereof.
[8] A pharmaceutical composition comprising, as an active ingredient, a compound of any of the above-mentioned [1]-[7] or a pharmaceutically acceptable acid addition salt thereof.
[9] The pharmaceutical composition of the above-mentioned
[8] to be used for the prophylaxis and/or treatment of osteoporosis, climacteric syndrome or breast cancer.
[10] Use of a compound of any of the above-mentioned [1]-[7] or a pharmaceutically acceptable acid addition salt thereof for the prophylaxis or treatment of osteoporosis, climacteric syndrome or breast cancer.
[11] A method for the prophylaxis or treatment of osteoporosis, climacteric syndrome or breast cancer, which comprises administering an effective amount of a compound of any of the above-mentioned [1]-[7] or a pharmaceutically acceptable acid addition salt thereof to a patient with osteoporosis, climacteric syndrome or breast cancer.
[12] A commercial package comprising the pharmaceutical composition of the above-mentioned [8] or [9] and a written matter associated therewith, the written matter stating that the pharmaceutical composition can or should be used for the prophylaxis or treatment of osteoporosis, climacteric syndrome or breast cancer.
[13] A spiro compound represented by the following formula (II): 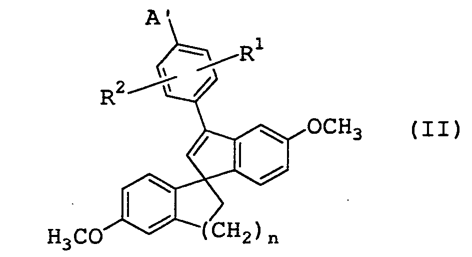 wherein R¹ and R² are the same or different and each is a hydrogen atom, a fluorine atom, a chlorine atom or a C₁₋₆ alkyl group,
   n is 1, 2 or 3,
   A' is -OH; -OCH₂CH=CH₂; -OSO₂CF₃; -(CH₂)ₚ-N(R³)(R⁴); -X-(CH₂)_{q}-N(R³)(R⁴); a group represented by the following formula (a) 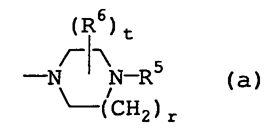 a group represented by the following formula (b) 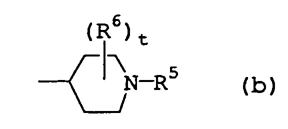 a group represented by the following formula (c) 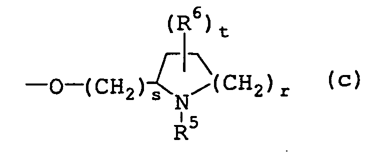 wherein p is 1, 2 or 3,
   X is an oxygen atom or a sulfur atom,
   q is 2 or 3,
   R³ and R⁴ are the same or different and each is a C₁₋₆ alkyl group or a phenyl C₁₋₄ alkyl group, or R³ and R⁴ optionally form, together with the adjacent nitrogen atom, a pyrrolidine ring, a piperidine ring, a homopiperidine ring, a piperazine ring or a morpholine ring, each optionally substituted by one or two groups selected from C₁₋₆ alkyl, phenyl and benzyl,
   R⁵ is a C₁₋₆ alkyl group, a C₃₋₈ cycloalkyl group or a C₃₋₈ cycloalkyl C₁₋₄ alkyl group,
   R⁶ is a hydrogen atom or a C₁₋₄ alkyl group, and
   r, s and t are each independently one or two.
[14] A spiro compound represented by the following formula (III): 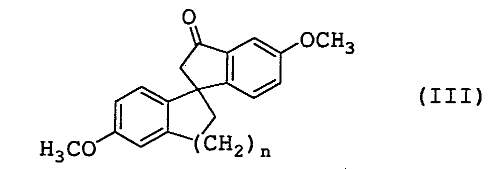
wherein n is 1, 2 or 3.

The present invention aims at providing a spiro compound useful as a SERM. In particular, the present invention aims at providing a compound highly useful for the prophylaxis and/or treatment of osteoporosis, climacteric syndrome or breast cancer. In addition, the present invention aims at providing a pharmaceutical composition comprising the compound. Furthermore, the present invention aims at providing an intermediate for producing the compound. These objects and other objects and advantages will be clear for those of ordinary skill in the art from the following description.

### DETAILED DESCRIPTION OF THE INVENTION

According to the present invention, a spiro compound represented by the following formula (I) and a pharmaceutically acceptable acid addition salt thereof (hereinafter sometimes to be also referred to as "the compound of the present invention") are provided. 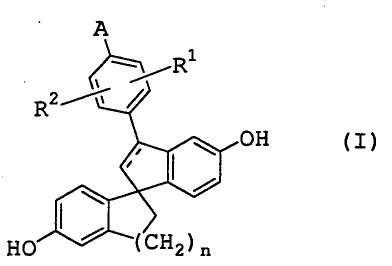 wherein R¹ and R² are the same or different and each is a hydrogen atom, a fluorine atom, a chlorine atom or a C₁₋₆ alkyl group,
n is 1, 2 or 3,
a bond containing a broken line is a single bond or a double bond,
A is -(CH₂)ₚ-N(R³)(R⁴); -X-(CH₂)_{q}-N(R³)(R⁴); a group represented by the following formula (a) 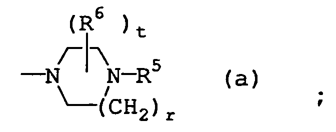 a group represented by the following formula (b) 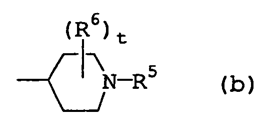 or a group represented by the following formula (c) 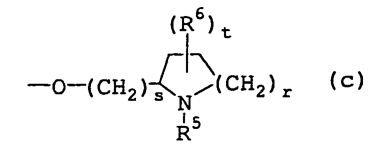 wherein p is 1, 2 or 3,
X is an oxygen atom or a sulfur atom,
q is 2 or 3,
R³ and R⁴ are the same or different and each is a C₁₋₆ alkyl group or a phenyl C₁₋₄ alkyl group, or R³ and R⁴ optionally form, together with the adjacent nitrogen atom, a pyrrolidine ring, a piperidine ring, a homopiperidine ring, a piperazine ring or a morpholine ring, each optionally substituted by one or two groups selected from a C₁₋₆ alkyl, phenyl and benzyl,
R⁵ is a C₁₋₆ alkyl group, a C₃₋₈ cycloalkyl group or a C₃₋₈ cycloalkyl C₁₋₄ alkyl group,
R⁶ is a hydrogen atom or a C₁₋₄ alkyl group and
r, s and t are each independently one or two.

As a pharmaceutically acceptable salt of the compound represented by the formula (I), for example, inorganic acid salts such as hydrochloride, hydrobromide, hydroiodide, sulfate, phosphate and the like, organic acid salts such as oxalate, malonate, maleate, fumarate, lactate, malate, citrate, tartrate, benzoate, trifluoroacetate, acetate, methanesulfonate, p-toluenesulfonate, trifluoromethanesulfonate and the like, amino acid salts such as glutamate and aspartate and the like can be mentioned.

Since the compound of the formula (I) and an acid addition salt thereof may be present in the form of a hydrate and/or a solvate, these hydrates and/or solvates are also encompassed in the compound of the present invention.

The spiro carbon atom of the compound of the formula (I) is an asymmetric carbon atom. In addition, the compound of the formula (I) sometimes has one or more asymmetric carbon atoms. Furthermore, the compound of the formula (I) sometimes shows a geometric isomerism. Therefore, the compound of the formula (I) can exist as several kinds of steric isomers (e.g., optically active form, diastereoisomer and the like). These steric isomers, a mixture and a racemate thereof are encompassed in the compound of the present invention.

Since the compound of the present invention forms a spiro bond at the 1-position of indan or indene, a location number is afforded as shown in the following formula. In the present specification, the compound of the present invention is named according to this location number: 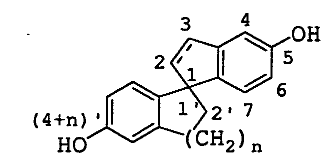 wherein n is 1, 2 or 3.

The terms in the present specification are explained below.

The "C₁₋₆ alkyl group" may be linear or branched chain, and specific examples thereof include methyl group, ethyl group, propyl group, isopropyl group, butyl group, isobutyl group, sec-butyl group, tert-butyl group, pentyl group, isopentyl group, neopentyl group, hexyl group, 2-ethylbutyl group and equivalents thereof. The "C₁₋₄ alkyl group" means methyl group, ethyl group, propyl group, isopropyl group, butyl group, isobutyl group, sec-butyl group or tert-butyl group.

Specific examples of the "C₃₋₈ cycloalkyl group" include a cyclopropyl group, a cyclobutyl group, a cyclopentyl group, a cyclohexyl group, a cycloheptyl group and a cyclooctyl group.

The "C₃₋₈ cycloalkyl C₁₋₄ alkyl group" means a "C₁₋₄ alkyl group" substituted by a "C₃₋₈ cycloalkyl group", and, for example, a cyclopropylmethyl group, a cyclopentylmethyl group, a cyclohexylmethyl group and equivalents thereof can be mentioned.

The "phenyl C₁₋₄ alkyl group" means a "C₁₋₄ alkyl group" substituted by a phenyl group, and, for example, a benzyl group, a phenethyl group, a phenylpropyl group and equivalents thereof can be mentioned.

A different name of "homopiperidine" is hexamethylenimine or hexahydro-1H-azepine.

The compound of the formula (I) wherein the bond containing a broken line is a single bond means a compound represented by the following formula (I-1) 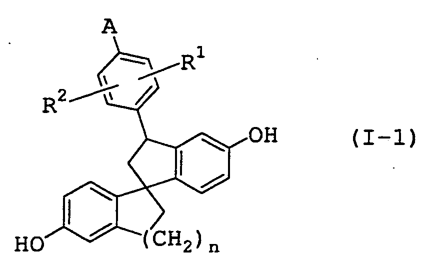 wherein R¹, R², n and A are as defined above and a bond between the 2-position and the 3-position is a single bond, and the compound of the formula (I) wherein the bond containing a broken line is a double bond means a compound represented by the following formula (I-2) 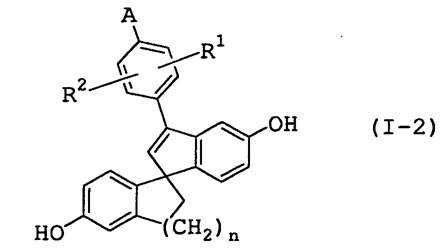 wherein R¹, R², n and A are as defined above and a bond between the 2-position and the 3-position is a double bond. A compound of the formula (I) wherein the bond containing a broken line is a double bond is preferable.

Of the compounds of the present invention, a compound that shows a superior activity as a SERM is a spiro compound of the formula (I) wherein A is -X-(CH₂)_{q}-N(R³)(R⁴) or a group represented by the following formula (a') 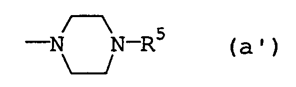 wherein R¹, R², R³, R⁴, R⁵, X, n, q and a bond containing a broken line are as defined above, or a pharmaceutically acceptable acid addition salt thereof.

A spiro compound of the formula (I) wherein A is - X-(CH₂)_{q}-N(R³)(R⁴) and R¹, R², R³, R⁴, X, n, q and a bond containing a broken line are as defined above, and a pharmaceutically acceptable acid addition salt thereof are more preferable.

A spiro compound of the formula (I) wherein A is - O-(CH₂)_{q}-N(R³)(R⁴), n is 1 and R¹, R², R³, R⁴, q and a bond containing a broken line are as defined above and a pharmaceutically acceptable acid addition salt thereof are still more preferable.

Of the compounds of the present invention, a still more superior compound is a spiro compound of the formula (I), wherein R¹ and R² are the same or different and each is a hydrogen atom, a fluorine atom or a chlorine atom, A is -O-(CH₂)₂-N(R³⁰)(R⁴⁰), n is 1, R³⁰ and R⁴⁰ are the same or different and each is a C₁₋₆ alkyl group, or R³⁰ and R⁴⁰ optionally form, together with the adjacent nitrogen atom, a pyrrolidine ring, a piperidine ring of a homopiperidine ring, each optionally substituted by one or two C₁₋₃ alkyl, a bond containing a broken line is a single bond or a double bond, or a pharmaceutically acceptable acid addition salt thereof.

A particularly preferable compound is a spiro compound represented by the following formula (I-1a) or (I-2a) or a pharmaceutically acceptable acid addition salt thereof: 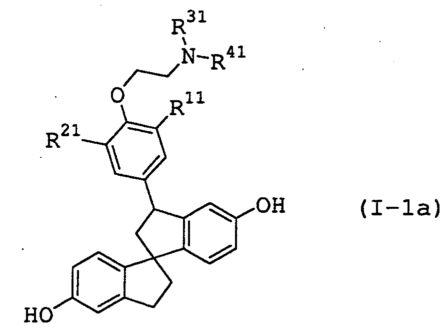 wherein R¹¹ and R²¹ are the same or different and each is a hydrogen atom, a fluorine atom or a chlorine atom,
R³¹ and R⁴¹ form, together with the adjacent nitrogen atom, a pyrrolidine ring, a piperidine ring or a homopiperidine ring, each optionally substituted by one or two methyl groups: 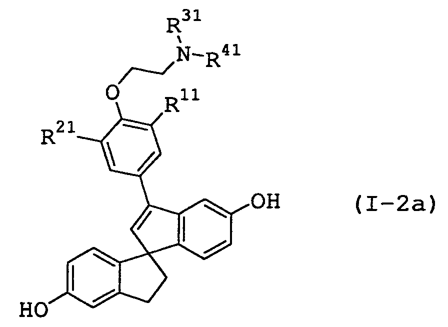 wherein R¹¹, R²¹, R³¹ and R⁴¹ are as defined above.

Specific examples of the particularly preferable compound include the following spiro compounds:
(1R*,3S*)-3-[4-(2-piperidinoethoxy)phenyl]-1,1'-spirobiindan-5,5'-diol (compound of Example 36A),
3-[4-(2-piperidinoethoxy)phenyl]spiro[indene-1,1'-indan]-5,5'-diol (compound of Example 1),
(+)-3-[4-(2-piperidinoethoxy)phenyl]spiro[indene-1,1'-indan]-5,5'-diol (compound of Example 37), and
(-)-3-[4-(2-piperidinoethoxy)phenyl]spiro[indene-1,1'-indan]-5,5'-diol (compound of Example 38), and pharmaceutically acceptable acid addition salts thereof.

A spiro compound represented by the following formula (II) or (III) is useful as an intermediate: 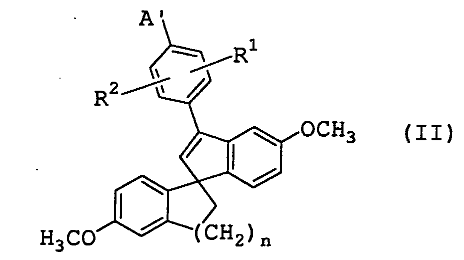 wherein R¹ and R² are the same or different and each is a hydrogen atom, a fluorine atom, a chlorine atom or a C₁₋₆ alkyl group,
n is 1, 2 or 3,
A' is -OH; -OCH₂CH=CH₂; -OSO₂CF₃; -(CH₂)ₚ-N(R³)(R⁴); -X-(CH₂)_{q}-N(R³)(R⁴); a group represented by the following formula (a) 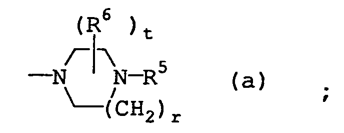 a group represented by the following formula (b) 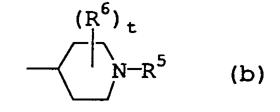 or a group represented by the following formula (c) 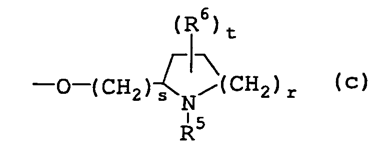 wherein p is 1, 2 or 3,
X is an oxygen atom or a sulfur atom,
q is 2 or 3,
R³ and R⁴ are the same or different and each is a C₁₋₆ alkyl group or a phenyl C₁₋₄ alkyl group, or R³ and R⁴ optionally form, together with the adjacent nitrogen atom, a pyrrolidine ring, a piperidine ring, a homopiperidine ring, a piperazine ring or a morpholine ring, each optionally substituted by one or two groups selected from a C₁₋₆ alkyl, phenyl and benzyl,
R⁵ is a C₁₋₆ alkyl group, a C₃₋₈ cycloalkyl group or a C₃₋₈ cycloalkyl C₁₋₄ alkyl group,
R⁶ is a hydrogen atom or a C₁₋₄ alkyl group,
r, s and t are each independently one or two: 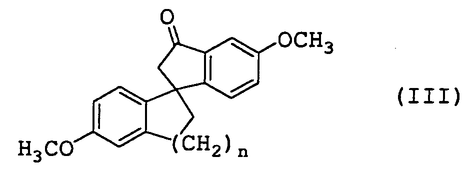 wherein n is 1, 2 or 3.

The compound of the formula (I) can be produced by, for example, the following production method

### Production method (A1)

A compound of the formula (I) wherein a bond containing a broken line is a single bond, i.e., a compound of the aforementioned formula (I-1) can be produced by hydrogenation of a compound of the formula (I) wherein a bond containing a broken line is a double bond, i.e., a compound of the aforementioned formula (I-2).

The hydrogenation reaction is carried out by reacting a compound of the formula (I-2) with hydrogen in a suitable solvent in the presence of a catalyst at normal pressure or under pressurization. As the catalyst, for example, palladium on carbon, palladium hydroxide on carbon, platinum, rhodium on carbon and the like can be mentioned. As the solvent, for example, alcohols such as methanol, ethanol, isopropanol and butanol, ethyl acetate, dimethylformamide, N-methylpiperidone, acetic acid can be mentioned. The reaction temperature is generally 0°C-60°C.

When a diastereomer is produced by this production method, it is isolated and purified by a conventional method such as chromatography, recrystallization, reprecipitation and the like.

### Production method (A2)

A compound of the formula (I) wherein a bond containing a broken line is a double bond, i.e., a compound of the aforementioned formula (I-2), can be produced by demethylation of a compound represented by the following formula (II-1) 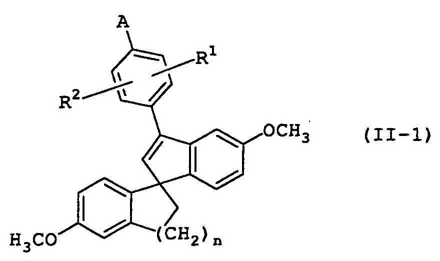 wherein R¹, R², n and A are as defined above.

This demethylation is performed by reacting a compound of the formula (II-1) with lithium diphenylphosphine in a suitable solvent. Instead of lithium diphenylphosphine, boron tribromide, sodium ethylthiolate, pyridine/hydrogen chloride complex, ethanethiol/aluminum chloride and the like can be used. As the solvent to be used, for example, ethers such as dimethoxyethane, diglyme, tetrahydrofuran, 1,4-dioxane and the like, hydrocarbons such as toluene, xylene and the like, organic halogens such as chloroform, dichloromethane, 1,2-dichloroethane, carbon tetrachloride and the like, dimethylformamide and N-methylpiperidone can be mentioned. These solvents are used alone, or as a mixed solvent of two or more kinds thereof. The reaction temperature is generally 0°C-200°C, preferably 20°C-100°C.

### Production method (B1) of intermediate

The compound of the aforementioned formula (II-1) can be produced by reacting a compound of the aforementioned formula (III) with a compound represented by the following formula (IV-1) 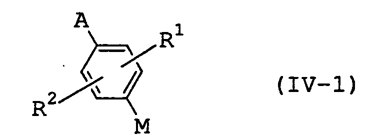 wherein M is Li or MgBr and R¹, R² and A are as defined above, in a suitable solvent reaction, and then dehydrating in the presence of an acid catalyst in a suitable solvent.

As the solvent for this reaction, for example, diethyl ether, tetrahydrofuran, toluene, xylene, 1,2-dichloroethane can be used as appropriate. These solvents are used alone, or as a mixture of two or more kinds thereof. As the acid catalyst, for example, p-toluenesulfonic acid, camphorsulfonic acid, trifluoroacetic acid, methanesulfonic acid, trifluoromethanesulfonic acid, sulfuric acid and hydrochloric acid can be mentioned. The dehydrating reaction proceeds generally at 0°C-150°C, preferably 80°C-120°C.

The compound of the formula (IV-1) can be produced by reacting a compound represented by the following formula (IV-2) 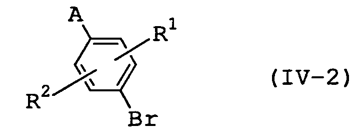 wherein R¹, R² and A are as defined above, with alkyl lithium or metal magnesium. As the alkyl lithium, for example, n-butyl lithium, sec-butyl lithium and tert-butyl lithium can be mentioned.

The reaction of a compound of the formula (IV-2) with alkyl lithium and the reaction of a compound of the formula (IV-1) with a compound of the formula (III) generally proceeds at -100°C to 120°C, preferably -80°C to 0°C. As the solvent, for example, tetrahydrofuran, toluene and diethyl ether can be used as appropriate. The reaction of the compound of the formula (IV-2) with metal magnesium can be carried out according to conventional methods of Grignard reaction. Specific embodiments are shown in Examples D-F.

### Production method (B2) of intermediate

A compound of the aforementioned formula (III) can be produced from a compound of the following formula (V), which is commercially available or known *per se,* by a method shown in the following: 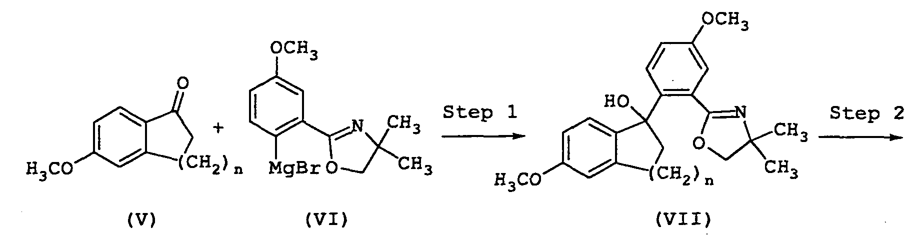 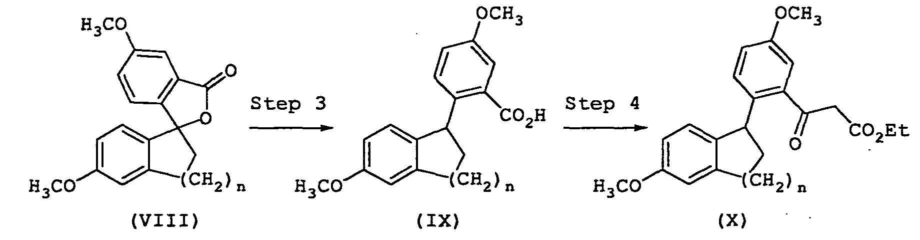 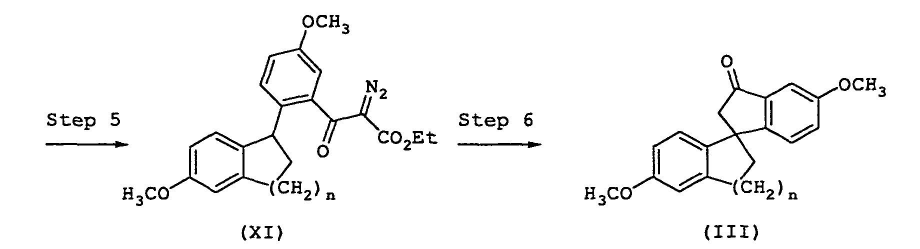 wherein n is as defined above.
Step 1: The compound of the above-mentioned formula (VII) can be produced by reacting a compound of the formula (V) with a compound of the formula (VI) under the conventional reaction conditions for Grignard reaction.

The compound of the formula (VI) can be produced according to the methods described in J. Med. Chem., 19, 1315 (1976) and J. Org. Chem., 40, 1427 (1975), using 2-(2-bromo-5-methoxyphenyl)-4,4-dimethyl-4,5-dihydrooxazole, which is commercially available or known per se, as a starting material.
Step 2: The compound of the above-mentioned formula (VIII) can be produced by reacting a compound of the formula (VII) with an aqueous sulfuric acid solution in a suitable solvent, preferably an ether solvent such as 1,4-dioxane and diglyme.
Step 3: The compound of the above-mentioned formula (IX) can be produced by subjecting a compound of the formula (VIII) to a hydrogenation reaction in the same manner as in the aforementioned Production method (A1).
Step 4: The compound of the above-mentioned formula (X) can be produced according to a method generally employed for producing β-ketoesters from carboxylic acids using a compound of the formula (IX) as a starting material.
Step 5: The compound of the above-mentioned formula (XI) can be produced by diazotization of the α-position of β-ketoester according to conventional methods, using a compound of the formula (X) as a starting material.

Specific embodiments of the above-mentioned Steps 1-5 are shown in Reference Example 1 and Reference Example 2 to be mentioned later. Step 6: the compound of the above-mentioned formula (III) can be produced according to the method described in J. Amer. Chem. Soc., 107, 196 (1985) by reacting a compound of the formula (XI) with rhodium acetate(II). Specific embodiments are shown in Examples A-C.

The compound of the formula (IV-2) is a commercially available compound, or can be produced by a method known *per se,* the method described in Production methods (C1)-(C4) to be mentioned later, or according to these methods.

A compound of the aforementioned formula (III), wherein n is 1 can be also produced by oxidizing 5,5'-dimethoxyspirobi-1,1'-indan according to the method described in Tetrahedron Lett., 28, 3131 (1987). 5,5'-Dimethoxyspirobi-1,1'-indan can be produced by a method described in Bull. Chem. Soc. Jpn., 44, 496 (1971).

### Production method (B3) of intermediate

A compound of the formula (II-1), wherein A is -O-(CH₂)_{q}-N(R³)(R⁴) or a group represented by the aforementioned formula (c) can be produced by dehydrating condensation of a compound represented by the following formula (II-2) 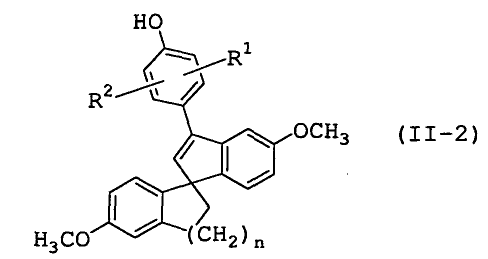 wherein R¹, R² and n are as defined above, with a compound represented by the following formula (XII)

HO-(CH₂)_{q}-N(R³)(R⁴) (XII)

wherein R³, R⁴ and q are as defined above, or a compound represented by the following formula (XIII) 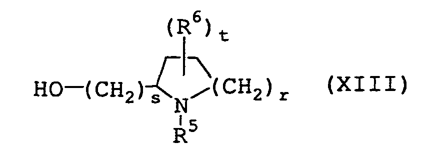 wherein R⁵, R⁶, r, s and t are as defined above.

The dehydrating condensation can be accomplished by the Mitsunobu reaction, namely, by reacting a compound of the formula (II-2), a compound of the formula (XII) or a compound of the formula (XIII), triphenylphosphine and azodicarboxylates. This reaction can be carried out according to the conventional methods of Mitsunobu reaction, or a method according thereto. Specific embodiments are shown in Example L and Example M.

The compound of the above-mentioned formula (II-2) can be produced by removing of an allyl group of a compound of the following formula (II-3) according to conventional methods. Specific embodiments are shown in Examples H and K. 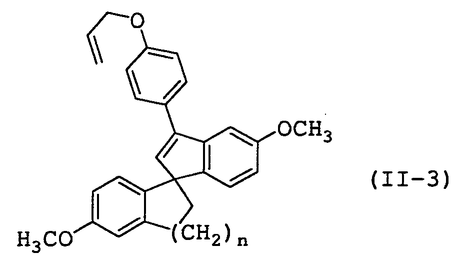 wherein n is as defined above.)

The compound of the above-mentioned formula (II-3) can be produced according to the method described in production method (B1) of intermediate, using a compound of the formula (III) and p-allyloxybromobenzene as starting materials. Specific embodiment is shown in Example G.

The compounds of the formulas (XII) and (XIII) are commercially available or can be synthesized by a method known per se.

### Production method (B4) of intermediate

A compound of the formula (II-1), wherein A is a group represented by the aforementioned formula (a) can be produced by reacting a compound represented by the following formula (II-4) 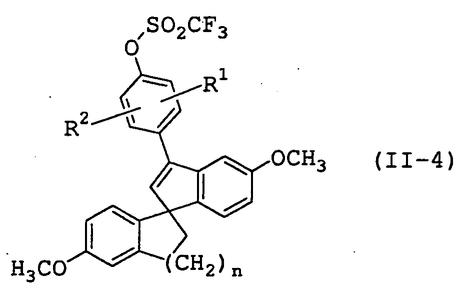 wherein R¹, R² and n are as defined above, with a compound represented by the following formula (XIV) 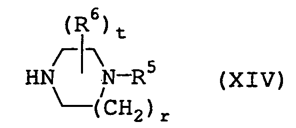 wherein R⁵, R⁶, r and t are as defined above.

The reaction between a compound of the formula (II-4) and a compound of the formula (XIV) is carried out in the presence of a palladium catalyst, a phosphor ligand and a base under dry conditions. As the solvent to be used, for example, 1,4-dioxane, tetrahydrofuran, toluene, dimethylformamide and the like can be mentioned. As the palladium catalyst, palladium acetate or palladium chloride is used and as the phosphor ligand, 2-(di-tert-butylphosphino)biphenyl, diphenylphosphinoferrocene, 2-(diphenylphosphino)-1,1'-binaphthyl and the like are used. As the base, potassium phosphate, sodium tert-butoxide and cesium carbonate can be used. The reaction temperature is generally 60°C-150°C.

The compound of the formula (II-4) can be produced by reacting a compound of the aforementioned formula (II-2) with 2-[N,N-bis(trifluoromethylsulfonyl)amino]-5-chloropyridine, trifluoromethanesulfonyl chloride or trifluoromethanesulfonic anhydride, in the presence of a base in a suitable solvent. As the base to be used, for example, sodium hydride, triethylamine, potassium carbonate, sodium carbonate, pyridine, 2,6-lutidine can be mentioned. As the solvent, for example, toluene, xylene, dimethoxyethane, 1,2-dichloroethane, acetone, methyl ethyl ketone, 1,4-dioxane, diglyme, ethyl acetate, dimethylformamide, dimethyl sulfoxide and pyridine can be mentioned. The reaction temperature is generally -100°C to 150°C, preferably -20°C to 70°C. Specific embodiment is shown in Example N.

The compound of the formula (XIV) is commercially available or can be synthesized by a method known per se.

### Production method (C1) of compound of formula (IV-2)

A compound of the formula (IV-2), wherein A is -X-(CH₂)_{q}-N(R³)(R⁴), namely, the compound of the following formula (IV-2a) can be produced by a method shown in the following: 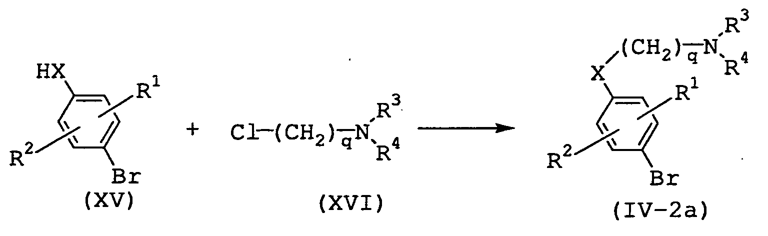 wherein R¹, R², R³, R⁴, X and q are as defined above.

The compound of the above-mentioned formula (XV) is reacted with compound of the above-mentioned formula (XVI) in the presence of a base in a suitable solvent. As the base, for example, sodium hydride, sodium hydroxide, potassium hydroxide and the like are used, and as the solvent, for example, dimethyl sulfoxide, dimethylformamide, toluene, xylene, 2-propanol and the like can be used as appropriate. The reaction temperature is generally 0-150°C, preferably 60-120°C. The compounds of the formula (XV) and the formula (XVI) are commercially available or can be synthesized by a method known per se.

### Production method (C2) of compound of formula (IV-2)

A compound of the formula (IV-2), wherein A is - (CH₂)ₚ-N(R³)(R⁴), namely, a compound of the following formula (IV-2b) can be produced by a method shown in the following: 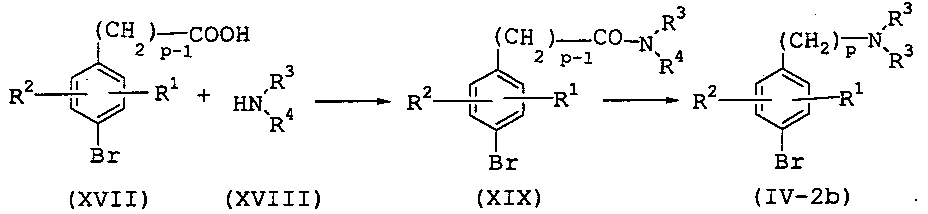 wherein R¹, R², R³, R⁴ and p are as defined above.

The compound of the above-mentioned formula (XIX) can be produced by condensing carboxylic acid represented by the formula (XVII) with a secondary amine represented by the formula (XVIII) in the presence of a dehydrating condensation agent in a suitable solvent, or, after conversion of a compound of the formula (XVII) to the corresponding acid chloride by a conventional method, condensation reaction of this compound with a compound of the formula (XVIII) in the presence of a base in a suitable solvent. As the dehydrating condensation agent, for example, N,N'-dicyclohexylcarbodiimide, 1-ethyl-3-(3-dimethylaminopropyl)carbodiimide hydrochloride, N,N'-carbonyldiimidazole, N,N'-carbonyldisuccinimide, 1-ethoxycarbonyl-2-ethoxy-1,2-dihydroquinoline, diphenylphosphoryl azide, propanephosphonic anhydride or benzotriazol-1-yloxy-tris(dimethylamino)phosphonium· hexafluorophosphate is used. As the base, for example, pyridine, sodium hydroxide, potassium carbonate, sodium hydrogen carbonate and the like are used. As the solvent, for example, dichloromethane, tetrahydrofuran, water, toluene and the like can be used as appropriate. The reaction temperature is generally -50°C to 150°C, preferably -10°C to 40°C.

The compound of the formula (IV-2b) can be produced by reacting a compound of the formula (XIX) with a borane·tetrahydrofuran complex in tetrahydrofuran. The reaction temperature is generally -50°C to 150°C, preferably 0°C to 80°C.

The compound of the formula (XVII) and the compound of the formula (XVIII) are commercially available or can be synthesized by a method known *per se.*

### Production method (C3) of compound of formula (IV-2)

The compound of the following formula (IV-2c) can be produced by subjecting a compound of the formula (XX) and a compound of the formula (XXI), which are obtained by production methods known *per se* or according to such methods, to the following route according to a method described in Production method (C2). Specific embodiments are shown in Reference Examples 3-5. 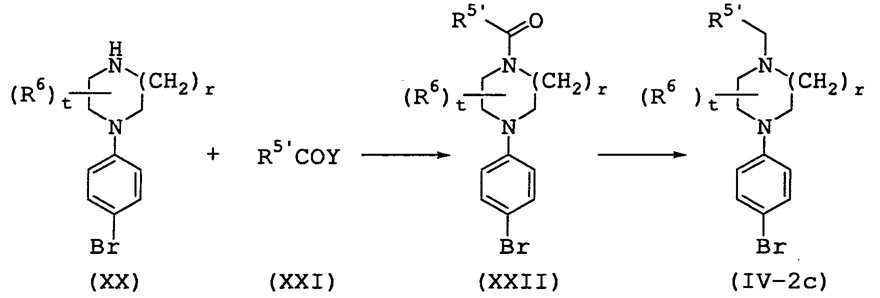 wherein R⁵' is a C₂₋₆ alkyl group or a C₃₋₈ cycloalkyl group, Y is a hydroxyl group or a halogen atom and R⁶, r and t are as defined above.

### Production method (C4) of compound of formula (IV-2)

The compound of the following formula (IV-2d) can be produced by reacting a compound of the following formula (XXV) with a borane·tetrahydrofuran complex in tetrahydrofuran. The compound of the formula (XXV) can be produced by reacting a compound of the formula (XXIII) and a compound of the formula (XXIV), which are obtained by production methods known per se or according to such methods, according to conventional methods. Specific embodiments are shown in Reference Examples 6 and 7. 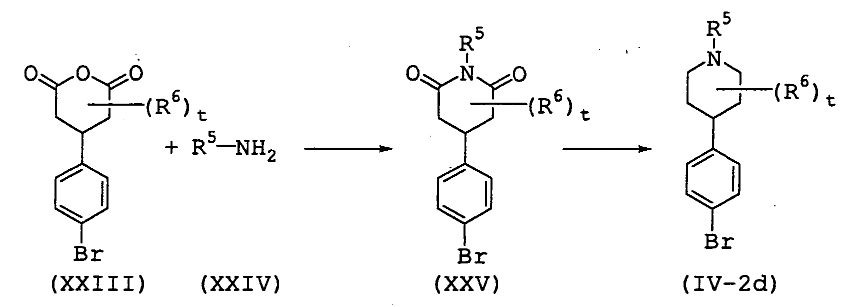 wherein R⁵, R⁶ and t are as defined above.

The compounds of the formula (I) produced by the aforementioned respective production methods can be isolated or purified by a conventional method such as chromatography, recrystallization, reprecipitation and the like. The compound of the formula (I) which is obtained in the form of a free base or an acid addition salt, depending on the kind of the functional group present in the structural formulas, determination of the starting compound and reaction treatment conditions, can be converted to a compound of the formula (I) according to conventional methods. The compound of the formula (I) can be led to an acid addition salt by treating with various acids according to conventional methods. In addition, various steric isomers of the compound of the formula (I) can be separated and purified according to conventional methods such as chromatography and the like.

Since the compounds of the formula (I-1), the formula (I-2), the formula (II-1), the formula (II-2), the formula (II-3) and the formula (III), obtained by the aforementioned respective production methods, are generally obtained as racemates, they can be separated and purified to give optically active forms thereof, according to conventional methods such as optical resolution method by chromatography using an optically active column, optical resolution method using a synthetic acidic agent or synthetic basic agent for chiral resolution, preferential crystallization method, diastereomer method and the like.

The pharmacological test results and pharmacological activity of the representative compounds of the present invention are explained in the following. The present invention is not limited by these Experimental Examples.

### Experimental Example 1a: Effects on climacteric syndrome (hot flash-like symptom)

The effect of the compound of the present invention on climacteric syndrome was investigated using ovariectomized rats. The ovariectomized rats were prepared by removing the ovary on the both sides from 8-week-old Jcl:SD female rats (manufactured by CLEA Japan, Inc.) under ether anesthesia. The rats were bred under the conditions of illumination for 12 hours (6:00-18:00) every day, room temperature 24±0.5°C, and were allowed free intake of solid feed (CE-2, manufactured by CLEA Japan, Inc.) and water. The rats after 2 weeks of ovariectomy were subjected to the following experiment. The rats to be used for sham surgery group were treated in the same manner except ovariectomy.

The rats after 2 weeks of ovariectomy and sham surgery were subjected to the measurement of tail skin temperature by reference to the method of Hosono et al. (Am. J. Physiol. Regulatory Integrative Comp. Physiol. 280: R1341-R1347, 2001) without anesthesia. To be specific, a temperature sensor (SXK-67, manufactured by TAKARA THERMISTOR) was fixed at 5 cm from the base of the tail of the rats with an adhesive plaster (manufactured by NICHIBAN), and covered with an aluminum plate. The other end of the temperature sensor was connected to a fluclet (manufactured by DAINIPPON PHARMACEUTICAL CO., LTD.) via an amplifier (E332, manufactured by TAKARA THERMISTOR) and measurement and analysis were performed for 2 hr. The results are shown in Table 1, wherein an incidence of a hot flash-like symptom was confirmed at the tail of the ovariectomized rats.

**Table 1**

| | Sham surgery group | Ovariectomy group |
|---|---|---|
| Average tail skin temperature (°C) | 29.1±0.4 | 31.6±0.4** |

| | | |
|---|---|---|
| ** <0.01: comparison with sham surgery group (Student's t-test) | | |

A test compound was dissolved in a solvent (10% aqueous cyclodextrin solution), and orally administered to ovariectomized rats once a day for two consecutive weeks. The tail skin temperature was measured in the same manner as in the aforementioned method on the day of the final administration. The results are shown in Table 2 and Table 3.

**Table 2**

| Test compound | Dose (mg/kg) | Average tail skin temperature (°C) |
|---|---|---|
| Solvent | - | 31.2±0.5 |
| β-estradiol | 3.0 10.0 | 28.4±0.6** 27.7±0.2** |
| | 0.1 | 29.4±0.3 |
| Compound of Example 1 | 1.0 10.0 | 28.9±0.4* 28.0±0.5** |
| | 0.1 | 29.9±0.4 |
| Compound of Example 36A | 1.0 10.0 | 29.4±0.4 29.0±0.4* |

| | | |
|---|---|---|
| *<0.05, | | |
| **<0.01: comparison with solvent control group (Dunnett's multiple comparison test) | | |

**Table 3**

| Test compound (dose; mg/kg) | Average tail skin temperature (°C) |
|---|---|
| Solvent | 31.0±0.6 |
| β-estradiol (3.0 mg) | 28.1±0.6** |
| β-estradiol (3.0 mg) + compound of Example 1 (10.0 mg) | 28.3±0.3^{a} |
| β-estradiol (3.0 mg) + compound of Example 36A (10.0 mg) | 28.8±0.7^{a} |

| | |
|---|---|
| **<0.01: Comparison with solvent control group (Student's t-test) | |
| a: no significant difference by comparison with β-estradiol (3.0 mg) | |

The compounds of Example 1 and Example 36A shown in Table 2 improved the hot flash-like symptom as did β-estradiol, which is an estrogen receptor agonist. The effect of the combined use of β-estradiol and the compound of the present invention shown in Table 3 was not significantly different from that of a single use of β-estradiol. In other words, the compounds of Example 1 and Example 36A did not inhibit the action of β-estradiol on the tissues involved in the hot flash-like symptom.

### Experimental Example 1b: luteinizing hormone (LH) level in serum

A test compound was dissolved in 10% aqueous cyclodextrin solution and orally administered to ovariectomized rats once a day for two consecutive weeks. The blood was drawn under ether anesthesia. The blood sample was allowed to coagulate at room temperature for 1 hr and centrifuged at 3000 rpm for 10 min to give serum. The amount of LH in serum was measured using a rat LH radioimmunoassay kit (manufactured by Immunotec Research Ltd., France). The results are shown in Table 4.

**Table 4**

| Rats used | Test compound | Dose (mg/kg) | Serum LH (ng/mL) |
|---|---|---|---|
| Sham surgery rat | Solvent | - | 0.10±0.03 |
| | Solvent | - | 14.3±0.80^{##} |
| Ovariectomized rat | β-estradiol Compound of Example 1 | 3.0 1.0 1.0 | 7.6±0.97** 10.1±0.60* 10.1±0.60* |

| | | | |
|---|---|---|---|
| ^{##} <0.01: Comparison with sham surgery rat·solvent control group (Student's t-test ) | | | |
| * <0.05, | | | |
| ** <0.01: Comparison with ovariectomized rat· solvent control group (Dunnett's multiple comparison test) | | | |

The compound of Example 1 shown in Table 4 significantly suppressed increase in the LH amount due to ovariectomy, as did β-estradiol. That is, the compound of Example 1 shows a preferable action on the hot flash-like symptom, which is a climacteric syndrome, as does estrogen.

### Experimental Example 2: Effect on decreased femur bone density of ovariectomized rat

The ovary on the both sides of 13-week-old Jcl:SD female rats (manufactured by CLEA Japan, Inc.) were removed under ether anesthesia or the rats were subjected to a sham surgery, and a test compound dissolved in 10% aqueous cyclodextrin solution was orally administrated from the next day once a day for 4 consecutive weeks (10 rats/group). The rats were sacrificed by hemorrhage on the next day of the final administration and the left femur was removed. The bone density of the femural metaphysis was measured using pQCT [peripheral Quantitative Computed Tomography, XCT-960A, manufactured by Norland·Stratec]. The results are shown in Table 5 (1) and Table 5(2) .

**Table 5 (1)**

| Rat used | Test compound | Dose (mg/kg) | Bone density (mg/cm³) |
|---|---|---|---|
| Sham surgery rat | Solvent Solvent | - | 799.9±41.2 799.9±41.2 |
| Ovariectomized rat | Solvent | - | 648.6±40.5^{##} |
| | β-estradiol | 1.0 | 767.3±63.9** |
| | Compound of Example 1 | 1.0 | 738.0±47.9** |

| | | | |
|---|---|---|---|
| ^{##} <0.01: Comparison with sham surgery rat·solvent control group (Student's t-test) | | | |
| ** <0.01: Comparison with ovariectomized rat·solvent control group (Dunnett's multiple comparison test) | | | |

**Table 5(2)**

| Rat used | Test compound | Dose (mg/kg) | Bone density (mg/cm³) |
|---|---|---|---|
| Sham surgery rat | Solvent - | - | 723±21 |
| Ovariectomized rat | Solvent | - | 518±10^{##} |
| | Example 37 | 0.01 | 551±10** |
| | | 0.1 | 620±15** |
| | | 1.0 | 658±17** |

| | | | |
|---|---|---|---|
| ^{##} <0.01: Comparison with sham surgery rat·solvent control group (Student's t-test) | | | |
| ** <0.01: Comparison with ovariectomized rat·solvent control group (Dunnett's multiple comparison test) | | | |

The compounds of Example 1 and Example 37 as shown in Table 5 (1) and Table 5(2), significantly suppressed the decrease in the bone density due to ovariectomy, as did β-estradiol.

### Experimental Example 3: Effect on serum cholesterol level in rat

A test compound was dissolved in 10% aqueous cyclodextrin solution, and orally administrated to 7-week-old Jcl:SD male rats (manufactured by CLEA Japan, Inc.) once a day for 4 consecutive days. The blood was drawn on the next day of the final administration, and plasma cholesterol was measured using a measurement kit (cholesterol CII-testwako, manufactured by Wako Pure Chemical Industries, Ltd.). The results are shown in Table 6 (1) and Table 6(2) .

**Table 6 (1)**

| Test compound | Dose (mg/kg) | Blood cholesterol (mg/dl) |
|---|---|---|
| Solvent | - | 79.3±7.1 |
| β-estradiol | 0.3 | 62.7±7.5 |
| | 1.0 | 42.8±6.1 |
| | 3.0 | 25.8±2.0** |
| Solvent | - | 75.1±2.3 |
| Compound of Example 1 | 0.03 | 63.1±4.8 |
| | 0.1 | 53.8±7.3 |
| | 0.3 | 41.6±5.2** |

| | | |
|---|---|---|
| ** <0.01: Comparison with solvent control group (Dunnett's multiple comparison test) | | |

**Table 6(2)**

| Test compound | Dose (mg/kg) | Blood cholesterol (mg/dl) |
|---|---|---|
| Solvent | - | 75.0±2.2 |
| Compound of Example 24 | 1.0 | 36.0±3.0** |
| Solvent | - | 73.0±3.9 |
| Compound of Example 30 | 1.0 | 23.6±2.2** |

| | | |
|---|---|---|
| ** <0.01: Comparison with solvent control group (Student's t-test) | | |

The compounds of Examples 1, 24 and 30, which are shown in Table 6 (1) and Table 6(2) showed a significant blood cholesterol lowering action at a lower dose than β-estradiol.

### Experimental Example 4 Uterus weight of ovariectomized rat

To investigate the side effects of the compound of the present invention, the test compound was orally administered consecutively to ovariectomized rats and sham surgery rats in the same manner as in Experimental Example 1b, the uterus was removed and weighed. The results are shown in Table 7 and Table 8.

**Table 7**

| Rat used | Test compound | Dose (mg/kg) | Uterus weight (mg) |
|---|---|---|---|
| Sham surgery rat | Solvent | - | 574.0±36.3 |
| Ovariectomized rat | Solvent | - | 137.9±8.5^{##} |
| | β-estradiol | 1.0 | 406.3±20.6** |
| | Compound of Example 1 | 1.0 | 178.6±9.3 |

| | | | |
|---|---|---|---|
| ^{##} <0.01: Comparison with sham surgery rat·solvent control group (Student's t-test ) | | | |
| ** <0.01: Comparison with ovariectomized rat·solvent control group (Dunnett's multiple comparison test) | | | |

**Table 8**

| Rat used | Test compound (dose; mg/kg) | Uterus weight (mg) |
|---|---|---|
| Ovariectomized rat | Solvent | 107.3±4.3 |
| | β-estradiol (3.0 mg) | 491.7±19.6^{##} |
| | β-estradiol (3.0 mg) + Compound of Example 1 (10.0 mg) | 217.5±5.8** |

| | | |
|---|---|---|
| ^{##} <0.01: Comparison with solvent control group (Student's t-test) | | |
| ** <0.01: Comparison with β-estradiol single administration group (Student's t-test) | | |

Unlike β-estradiol, the compound of Example 1 shown in Table 7 does not significantly increase the uterus weight of ovariectomized rat. As shown in Table 8, moreover, the compound of Example 1 significantly suppressed the uterus weight increasing action of β-estradiol. In other words, the compound of Example 1 does not show side effects in uterus.

As is clear from the above pharmacological test, the compound of the formula (I) improves the climacteric syndrome (hot flash-like symptom) observed in the ovariectomized rats, prevents decrease of femural bone density of ovariectomized rats, has a hypolipidemic action, and does not cause changes in the uterus weight of ovariectomized rats. Therefore, the compound is useful for the prophylaxis and/or treatment of diseases estrogen is involved, such as climacteric syndrome, osteoporosis, chondral degeneration, endometriosis, gynecomastia, obesity, hyperlipidemia, arteriosclerotic disease, incontinence, autoimmune disease, breast cancer, endometrioma, colon cancer, lung cancer, prostate cancer, dysmnesia, cognitive impairment, dementia and the like. In particular, the compound of the formula (I) is expected to be a drug for the prophylaxis and/or treatment of osteoporosis, climacteric syndrome and breast cancer.

The administration route of the compound of the formula (I) may be any of oral administration, parenteral administration, rectal administration and transdermal administration. While the dose varies depending on the administration method, conditions of patients, age of patients, mode of treatment (prophylaxis or therapy) and the like, it is generally 0.01-40 mg/kg/day, preferably 0.1-20 mg/kg/day.

The compound of the formula (I) is generally administered in the form of a pharmaceutical composition prepared by admixing with carriers for preparations. As the carriers for pharmaceutical composition, substances conventionally used in the field of preparations, which do not react with the compound of the formula (I) is used. Specifically, for example, lactose, inositol, glucose, mannitol, dextran, cyclodextrin, sorbitol, starch, partial α starch, sucrose, magnesium aluminometasilicate, synthetic aluminometasilicate, crystalline cellulose, sodium carboxymethyl cellulose, hydroxypropylstarch, calcium carboxymethyl cellulose, ion exchange resin, methyl cellulose, gelatin, gum arabic, hydroxypropyl cellulose, low substituted hydroxypropyl cellulose, hydroxypropylmethyl cellulose, polyvinylpyrrolidone, polyvinyl alcohol, alginic acid, sodium alginate, light silicic anhydride, magnesium stearate, talc, carboxyvinyl polymer, titanium oxide, sorbitan fatty acid ester, sodium lauryl sulfate, glycerol, glycerol fatty acid ester, purified lanolin, glycerogelatin, polysorbate, macrogol, vegetable oil, wax, propylene glycol, water, ethanol, polyoxyethylene hydrogenated castor oil (HCO), sodium chloride, sodium hydroxide, hydrochloric acid, sodium monohydrogen phosphate, sodium dihydrogen phosphate, citric acid, glutamic acid, benzyl alcohol, methyl paraoxybenzoate, ethyl paraoxybenzoate, white petrolatum, Plastibase, white bee wax, macrogol and the like can be mentioned.

As the dosage form, tablet, capsule, granule, powder, syrup, suspension, suppository, injection, ointment, adhesive agent and the like can be mentioned. These preparations are prepared according to conventional methods. For liquid preparation, the compound of the formula (I) may be dissolved or suspended in water or other suitable solvent when in use. In addition, tablets and granules may be coated by a well-known method. In the case of an injection, the compound of the formula (I) is dissolved in water. Where necessary, an isotonicity agent or dissolution aids may be used for dissolution, and pH regulators, buffers and preservatives may be added.

These preparations can contain the compound of the formula (I) in a proportion of not less than 0.01%, preferably 0.1-70%. These preparations may further contain other therapeutically effective components.

### BEST MODE FOR CARRING OUT THE INVENTION

The present invention is explained in more detail in the following by referring to Reference Examples and Examples, which are not to be construed as limitative. The compound was identified by elemental analysis, hydrogen nuclear magnetic resonance absorption spectrum (¹H-NMR) and the like. The assignment of the peak of ¹H-NMR is described using the following abbreviations for simplified expression.
J: coupling constant, s: singlet, d: doublet, dd: double doublet, t: triplet, seq: septuplet, m: multiplet, br: broad, brs: broad singlet, brd: broad doublet, brt: broad triplet.

The basic silica gel column chromatography used in Reference Examples and Production Examples was CHROMATOREX NH manufactured by Fuji Silysia Chemical Ltd.

### Reference Example 1

### Production of 5-methoxy-2-(5-methoxy-1-indanyl)benzoic acid:

(1) Magnesium (22.3 g) was suspended in tetrahydrofuran (400 mL) and 1/10 amount of a solution of 2-(2-bromo-5-methoxyphenyl)-4,4-dimethyl-4,5-dihydrooxazole (243 g) in tetrahydrofuran (2 L) was added thereto. A small amount of iodine was added and the reaction mixture was heated under reflux. After the completion of vigorous reaction, the rest of the oxazole solution was added with heating under reflux over 1 hr. The mixture was further heated under reflux for 2 hr after the dropwise addition. Thereto was added dropwise under ice-cooling a solution of 5-methoxy-1-indanone (118 g) in tetrahydrofuran (1 L) over 0.5 hr. The mixture was stirred at room temperature for one day, saturated aqueous ammonium chloride solution (400 mL) was added and the mixture was stirred. The organic layer was separated and washed with water and saturated brine. The organic layer was dried over sodium sulfate and concentrated under reduced pressure to give an oil.
(2) The above-mentioned oil was suspended in a mixture of 1,4-dioxane (1 L) and 6 mol/L sulfuric acid (500 mL), and the mixture was heated under reflux for 6 hr. The mixture was concentrated under reduced pressure, and the residue was extracted by adding ethyl acetate (1 L), hexane (500 mL) and water (1 L). The organic layer was dried over sodium sulfate, and concentrated under reduced pressure to give an oil.
(3) The aforementioned oil was dissolved in acetic acid (2 L), 20% palladium hydroxide on carbon (50 g) was added, and the mixture was hydrogenated at room temperature for 12 hr. The precipitated crystals were dissolved by adding chloroform (600 mL) to the reaction mixture and the mixture was filtered. The filtrate was collected and concentrated under reduced pressure. Ethanol (300 mL) was added to the residue and the precipitated crystals were collected by filtration and washed with ethanol to give the title compound (135 g).
   melting point: 180-181°C

### Reference Example 2

### Production of ethyl 2-diazo-3-[5-methoxy-2-(5-methoxy-1-indanyl)phenyl]-3-oxopropanoate:

(1) 5-Methoxy-2-(5-methoxy-1-indanyl)benzoic acid (83 g) was suspended in toluene (300 mL) and oxalyl chloride (33.2 mL) and dimethylformamide (0.1 mL) were added. The mixture was stirred at room temperature for 4 hrs. The reaction mixture was concentrated under reduced pressure to give an oil.
(2) A 20% solution (789 mL) of potassium hexamethyldisilazane in toluene was diluted with tetrahydrofuran (700 mL) and ethyl acetate (68 mL) was added dropwise at -78°C over 1 hr thereto, and the mixture was further stirred at the same temperature for 1 hr. A solution of the above-mentioned oil in tetrahydrofuran (300 mL) was added dropwise over 1 hr thereto, and the mixture was further stirred for 1 hr. A saturated aqueous ammonium chloride solution (600 mL) was added to quench the reaction, and the organic layer was separated. The organic layer was washed with water and saturated brine. The organic layer was dried over sodium sulfate and concentrated under reduced pressure to give an oil.
(3) The aforementioned oil and p-acetoaminobenzenesulfonyl azide (80 g) were dissolved in acetonitrile (600 mL), to this solution was added triethylamine (92 mL) over 1 hr and the mixture was stirred at room temperature for 2 hr. The reaction mixture was filtered and the collected precipitate was washed with a mixed solvent of ethyl acetate (25 mL) and hexane (25 mL). The filtrate and washing solution was combined and concentrated under reduced pressure. The residue was subjected to column chromatography (ethyl acetate/hexane) to give the title compound (106 g). melting point: 97-99°C

### Reference Example 3

### Production of 1-(4-bromophenyl)-4-ethylpiperazine

(1) To a solution of 1-(4-bromophenyl)piperazine (1.0 g) in ethyl acetate (20 mL) were added water (20 mL) and sodium hydrogen carbonate (0.6 g) and the mixture was stirred vigorously. Acetic anhydride (0.5 mL) was added and the mixture was stirred at room temperature for 2 hr. The organic layer was separated, washed successively with saturated aqueous sodium hydrogen carbonate solution and saturated brine, and dried over sodium sulfate. The organic layer was concentrated under reduced pressure to give an oil.
(2) The above-mentioned oil was dissolved in tetrahydrofuran (5 mL), a 2.0M solution (4.5 mL) of borane methylsulfide complex in tetrahydrofuran was added and the mixture was stirred at room temperature for one day. 20% Hydrochloric acid (10 mL) was added and the mixture was heated under reflux for 1 hr. Tetrahydrofuran was evaporated under reduced pressure. A 15% aqueous sodium hydroxide solution was added until the pH of the reaction mixture became 9, and the mixture was extracted with ethyl acetate (20 mL). The organic layer was concentrated under reduced pressure and the residue was subjected to column chromatography (ethyl acetate/hexane) to give the title compound (0.86 g). melting point: 84-85°C

### Reference Examples 4-5

The reaction and treatment were performed in the same manner as in Reference Example 3 using the corresponding starting compound to give the compounds of Reference Example 4 and Reference Example 5.

### (Reference Example 4)

### 1-(4-bromophenyl)-4-propylpiperazine:

melting point: 86-87°C

### (Reference Example 5)

### 1-(4-bromophenyl)-4-isobutylpiperazine:

melting point: 95-97°C

### Reference Example 6

### Production of 4-(4-bromophenyl)-1-ethylpiperidine

(1) (4-bromophenyl)-3,4,5-trihydro-2H-pyran-2,6-dione (3.0 g) was suspended in toluene (30 mL) and 70% aqueous ethylamine solution (1.0 g) was added thereto. The mixture was stirred at room temperature for one day. Ethyl acetate (100 mL) was added to the reaction mixture and washed successively with 10% hydrochloric acid (20 mL) and saturated brine (20 mL). The organic layer was concentrated under reduced pressure to give an oil. This oil was dissolved in toluene (50 mL) and p-toluenesulfonic acid (0.2 g) was added. The mixture was heated under reflux for 15 hr using a Dean-Stark trap. The reaction solution was washed successively with water (10 mL) and saturated brine (20 mL) and concentrated under reduced pressure to give an oil.
(2) The above-mentioned oil (2.6 g) was dissolved in tetrahydrofuran (20 mL) and a 10M solution (2.6 mL) of borane methylsulfide complex in tetrahydrofuran was added. The mixture was stirred with heating under reflux for one day. 20% Hydrochloric acid (10 mL) was added and the mixture was heated under reflux for 6 hr. Tetrahydrofuran was evaporated under reduced pressure. A 15% aqueous sodium hydroxide solution was added until the pH of the reaction mixture became 9, and the mixture was extracted with ethyl acetate (20 mL). The organic layer was concentrated under reduced pressure and the residue was subjected to basic silica gel column chromatography (ethyl acetate/hexane) to give the title compound (1.93 g).
   melting point: 33-34°C

### Reference Example 7

### Production of 4-(4-bromophenyl)-1-isobutylpiperidine

The reaction and treatment were performed in the same manner as in Reference Example 6 using the corresponding starting material compound to give the title compound.
melting point: 36-37°C

### Example A

### Production of 5,5'-dimethoxy-3-oxaspirobi-1,1'-indan (compound of the formula (III) wherein n=1)

A solution of ethyl 2-diazo-3-[5-methoxy-2-(5-methoxy-1-indanyl)phenyl]-3-oxopropanoate (106 g) in α,α,α-trifluorotoluene (700 mL) was added dropwise to a vigorously stirred suspension of rhodium acetate(II) hydrate (1.0 g) in α,α,α-trifluorotoluene (100 mL) at 60°C over 2 hr. After the dropwise addition, the mixture was further stirred at the same temperature for 0.5 hr. The reaction mixture was concentrated under reduced pressure and the residue was subjected to column chromatography (ethyl acetate/hexane) to give an oil. This oil was dissolved in 90% aqueous dimethyl sulfoxide solution (750 mL) and the mixture was heated at 150°C for 1 hr. After allowing to cool, the reaction mixture was added to water (1.5 L) and stirred, and the precipitated crystals were collected by filtration. The crystals were washed with water and hexane and dried to give the title compound (65 g).
melting point: 94-96°C

### Example B

### Production of 5,6'-dimethoxy-3-oxospiro[indan-1,1'-(1',2',3',4'-tetrahydronaphthalene)](compound of the formula (III) wherein n=2)

The title compound was synthesized from 6-methoxytetralone by a method similar to Reference Example 1, Reference Example 2 and Example A.
¹H-NMR (300 MHz, CDCl₃)δ 1.75-1.99 (2H, m), 2.02-2.28 (2H, m), 2.75-3.05 (4H, m), 3.75 (3H, s), 3.86 (3H, s), 7.0-7.2 (3H, m), 7.6-7.7 (3H, m).

### Example C

### Production of 5,7'-dimethoxy-3-oxospiro[indan-1,1'-(6',7',8',9'-tetrahydro-5H-benzocycloheptene)](compound of the formula (III) wherein n=3)

The title compound was synthesized from 7-methoxysuberenone by a method similar to Reference Example 1, Reference Example 2 and Example A.
melting point: 143-145°C

### Example D

### Production of 5,5'-dimethoxy-3-[4-(2-piperidinoethoxy)phenyl]spiro[indene-1,1'-indan]

(1) A solution of 1-bromo-4-(2-piperidinoethoxy)benzene (4.23 g) in tetrahydrofuran (20 mL) was cooled to -78°C and 1.6M solution (9.3 mL) of butyl lithium in hexane was added thereto. After stirring at the same temperature for 0.5 hr, a solution of 5,5'-dimethoxy-3-oxospirobi-1,1'-indan (2.0 g) in tetrahydrofuran (10 mL) was added. After stirring for 1 hr, saturated aqueous ammonium chloride solution (20 mL) was added and the organic layer was separated. The organic layer was washed with saturated brine, dried over sodium sulfate and concentrated under reduced pressure to give an oil.
(2) The above-mentioned oil was dissolved in toluene (50 mL) and p-toluenesulfonic acid (3.9 g) was added thereto. The mixture was heated under reflux for 0.5 hr. After allowing to cool, the reaction mixture was washed with saturated brine, and dried over sodium sulfate. The organic layer was concentrated under reduced pressure, and the residue was subjected to column chromatography (ethyl acetate/hexane) to give the title compound (2.0 g) as an oil.
   ¹H-NMR (300 MHz, CDCl₃)δ 1.4-1.7 (6H, m), 2.49 (2H, t, J = 6.6 Hz), 2.9-3.1 (4H, m), 2.81 (2H, t, J = 6.0 Hz), 3.1-3.4 (2H, m), 3.79 (3H, s), 3.82 (3H, s), 4.16(2H, t, J = 6.6 Hz), 6.48 (1H, s), 6.5-6.6 (2H, m), 6.71 (1H, dd, J = 8.1, 2.1 Hz), 6.89 (1H, brs), 6.97 (2H, m), 7.05-7.10 (2H, m), 7.54 (2H, m).

### Example E

### Production of 3-[4-(4-ethyl-1-piperazinyl)phenyl]-5,5'-dimethoxyspiro[indene-1,1'-indan]

The reaction and treatment were performed in the same manner as in Example D using 1-(4-bromophenyl)-4-ethylpiperazine instead of 1-bromo-4-(2-piperidinoethoxy)benzene of Example D to give the title compound.
¹H-NMR (300 MHz, CDCl₃)δ 1.46 (3H, t, J = 6.9 Hz), 2.4-2.6 (4H, m), 2.63 (4H, drt, J = 5.1 Hz), 3.1-3.3 (2H, m), 3.29 (4H, brt, J = 5.1 Hz), 3.78 (3H, s), 3.82 (3H, s), 6.47 (1H, s), 6.5-6.6 (2H, m), 6.71 (1H, dd, J = 2.4, 8.4 Hz), 6.88 (1H, brs), 7.00 (2H, d, J = 9.0 Hz), 7.06 (1H, d, J = 8.1 Hz), 7.11 (1H, d, J = 2.4 Hz), 7.52 (2H, d, J = 8.4 Hz).

### Example F

### Production of 3-[4-(4-isobutyl-1-piperazinyl)phenyl]-5,5'-dimethoxyspiro[indene-1,1'-indan]

The reaction and treatment were performed in the same manner as in Example D using 4-(4-bromophenyl)-1-isobutylpiperazine instead of 1-bromo-4-(2-piperidinoethoxy)benzene of Example D to give the title compound.
¹H-NMR (300 MHz, CDCl₃)δ 0.93 (6H, d, J = 6.6 Hz), 1.7-1.9 (4H, m), 1.9-2.1 (2H, m), 2.12 (2H, d, J = 7.2 Hz) 2.50 (2H, t, J = 6.9 Hz), 2.52 (1H, m), 3.01 (2H, brd, J = 11.2 Hz), 3.6-3.8 (2H, m), 3.78 (3H, s), 3.82 (3H, s), 6.53 (1H, s), 6.5-6.6 (2H, m), 6.71 (1H, dd, J = 2.4, 10.5 Hz), 6.88 (1H, brs), 7.07 (2H, d, J = 8.4 Hz), 7.11 (1H, d, J = 2.1 Hz), 7.31 (2H, d, J = 8.4 Hz), 7.55 (2H, d, J = 8.4 Hz).

### Example G

### Production of (+)-3-(4-allyloxyphenyl)-5,5'-dimethoxyspiro[indene-1,1'-indan] and (-)-3-(4-allyloxyphenyl)-5,5'-dimethoxyspiro[indene-1,1'-indan]

A part of a solution of p-allyloxybromobenzene (22.1 g) in tetrahydrofuran (100 mL) and a small amount of iodine were added to a suspension of magnesium (2.91 g) in tetrahydrofuran (100 mL) and the mixture was stirred. After a vigorous reaction ceased, the rest of the p-allyloxybromobenzene solution was added dropwise over 0.5 hr and, after dropwise addition of the entire amount, the mixture was further stirred for 0.5 hr. A solution of 5,5'-dimethoxy-3-oxospirobi-1,1'-indan (15 g) in tetrahydrofuran (50 mL) was added dropwise thereto at room temperature and the mixture was stirred for one day. A saturated ammonium chloride solution (50 mL) was added and, after stirring, tetrahydrofuran was evaporated under reduced pressure. The residue was extracted with ethyl acetate/water and the organic layer was washed with saturated brine and dried over sodium sulfate. The organic layer was concentrated under reduced pressure to give an oil. This oil was dissolved in toluene (150 mL) and p-toluenesulfonic acid (0.3 g) was added. A Dean-Stark trap was set and the mixture was heated under reflux for 2 hr. After allowing to cool, the mixture was extracted with ethyl acetate/water. The organic layer was washed with saturated brine, and dried over sodium sulfate. The organic layer was concentrated under reduced pressure, and the residue was subjected to column chromatography (ethyl acetate/hexane) to give 3-(4-allyloxyphenyl)-5,5'-dimethoxyspiro[indene-1,1'-indan] (racemic compound) (16.2 g) as an oil.

The above-mentioned racemic compound (11 g) was separated by high performance liquid chromatography (column; CHIRALCEL OJ, mobile phase; hexane/2-propanol=50/50, flow rate; 0.5mL/min) to give 4.9 g each of two kinds of the title optically active compounds at an optical purity of not less than 99% for both, retention time of [(+)-3-(4-allyloxyphenyl)-5,5'-dimethoxyspiro[indene-1,1'-indan] 17.27 min, retention time of (-)-3-(4-allyloxyphenyl)-5,5'-dimethoxyspiro[indene-1,1'-indan] 32.39 min].

### Example H

### Production of (+)-3-(4-hydroxyphenyl)-5,5'-dimethoxyspiro[indene-1,1'-indan]

(+)-3-(4-Allyloxyphenyl)-5,5'-dimethoxyspiro[indene-1,1'-indan] (0.5 g) was dissolved in benzene (10 mL) and palladium acetate (27 mg), triphenylphosphine (0.12 g) and formic acid (1 mL) were added thereto. The mixture was heated under reflux for 1 hr. The reaction mixture was extracted with an ethyl acetate/saturated sodium hydrogen carbonate solution.
The organic layer was washed with saturated brine, dried over sodium sulfate and concentrated under reduced pressure. The residue was subjected to column chromatography (ethyl acetate/hexane) to give the title compound (0.43 g) as an oil.
[α]_{D}²⁴ +82.6° (c 1.34,CHCl₃)

### Example K

### Production of (-)-3-(4-hydroxyphenyl)-5,5'-dimethoxyspiro[indene-1,1'-indan]

The reaction and treatment were performed in the same manner as in Example H to give the title compound from (-)-3-(4-allyloxyphenyl)-5,5'-dimethoxyspiro[indene-1,1'-indan].
[α]_{D}²⁴ +82.4° (c 1.42,CHCl₃)

### Example L

### (+)-5,5'-dimethoxy-3-[4-(2-piperidinoethoxy)phenyl]spiro[indene-1,1'-indan]

(+)-3-(4-Hydroxyphenyl)-5,5'-dimethoxyspiro[indene-1,1'-indan] (0.41 g), piperidinoethanol (0.3 g) and triphenylphosphine (0.61 g) were dissolved in anhydrous tetrahydrofuran (5 mL) and diisopropyl azodicarboxylate (0.46 mL) was gradually added under ice-cooling. After stirring at room temperature for one day, the mixture was concentrated under reduced pressure, and the residue was subjected to basic silica gel column chromatography (ethyl acetate/hexane) to give the title compound (0.41 g) as an oil.
[α]_{D}²⁴ +58.6° (c 0.56,CHCl₃)

### Example M

### (-)-5,5'-dimethoxy-3-[4-(2-piperidinoethoxy)phenyl]spiro[indene-1,1'-indan]

The reaction and treatment were performed in the same manner as in Example L to give the title compound from (-)-3-(4-hydroxyphenyl)-5,5'-dimethoxyspiro[indene-1,1'-indan].
[α]_{D}²⁴ -58.8° (c 0.72,CHCl₃)

### Example N

### Production of 3-(4-trifluoromethanesulfonyloxyphenyl)-5,5'-dimethoxyspiro[indene-1,1'-indan]

(+)-3-(4-Hydroxyphenyl)-5,5'-dimethoxyspiro[indene-1,1'-indan] (0.87 g) was dissolved in anhydrous dimethylformamide and 60% sodium hydride (0.1 g) was added thereto. The mixture was stirred at room temperature for 1 hr. 2-[N,N-bis(trifluoromethylsulfonyl)amino]-5-chloropyridine (1.0 g) was added, and the mixture was stirred for one day. The reaction mixture was extracted with ethyl acetate/10% hydrochloric acid and the organic layer was washed successively with water, 10% aqueous sodium hydroxide solution, water and saturated brine. The organic layer was dried over sodium sulfate and concentrated under reduced pressure, and the residue was subjected to column chromatography (ethyl acetate/hexane) to give the title compound (1.0 g) as an oil.
¹H-NMR (300 MHz, CDCl₃)δ 2.51 (2H, t, J = 7.5 Hz), 3.22 (2H, m), 3.79 (3H, s), 3.82 (3H, s), 6.5-6.6 (3H, m), 6.75 (1H, dd, J = 2.1, 8.1 Hz), 6.90 (1H, brs), 7.01 (1H, d, J = 2.4 Hz), 7.11 (1H, d, J = 8.4 Hz), 7.34 (2H, d, J = 9.0 Hz), 7.68 (2H, d, J = 9.0 Hz).

### Example 1

### Production of 3-[4-(2-piperidinoethoxy)phenyl]spiro[indene-1,1'-indan]-5,5'-diol hydrochloride

Diphenylphosphine (1.68 mL) was dissolved in tetrahydrofuran (20 mL) and the mixture was ice-cooled. A 1.6M solution (6.0 mL) of butyl lithium in hexane was added thereto and the mixture was stirred at the same temperature for 0.5 hr. To this reaction mixture was added a solution of 5,5'-dimethoxy-3-[4-(2-piperidinoethoxy)phenyl]spiro[indene-1,1'-indan] (0.58 g) in tetrahydrofuran (10 mL) and the mixture was heated under reflux for 20 hr. After allowing to cool, saturated ammonium chloride solution (20 mL) was added and the organic layer was separated. The organic layer was washed with saturated brine, dried over sodium sulfate and concentrated under reduced pressure. The obtained residue was subjected to basic silica column chromatography (chloroform/methanol) to give the title compound (0.37 g) as an oil. This was dissolved in water-containing methanol, hydrochloric acid was added and freeze-dried to give hydrochloride thereof.

The ¹H-NMR data of the title compound are shown in Table 9.

### Examples 2-21

The reaction and treatment were performed in the same manner as in Example D and Example 1 using the corresponding starting compound to give the compounds of Examples 2-21 shown in Table 9.

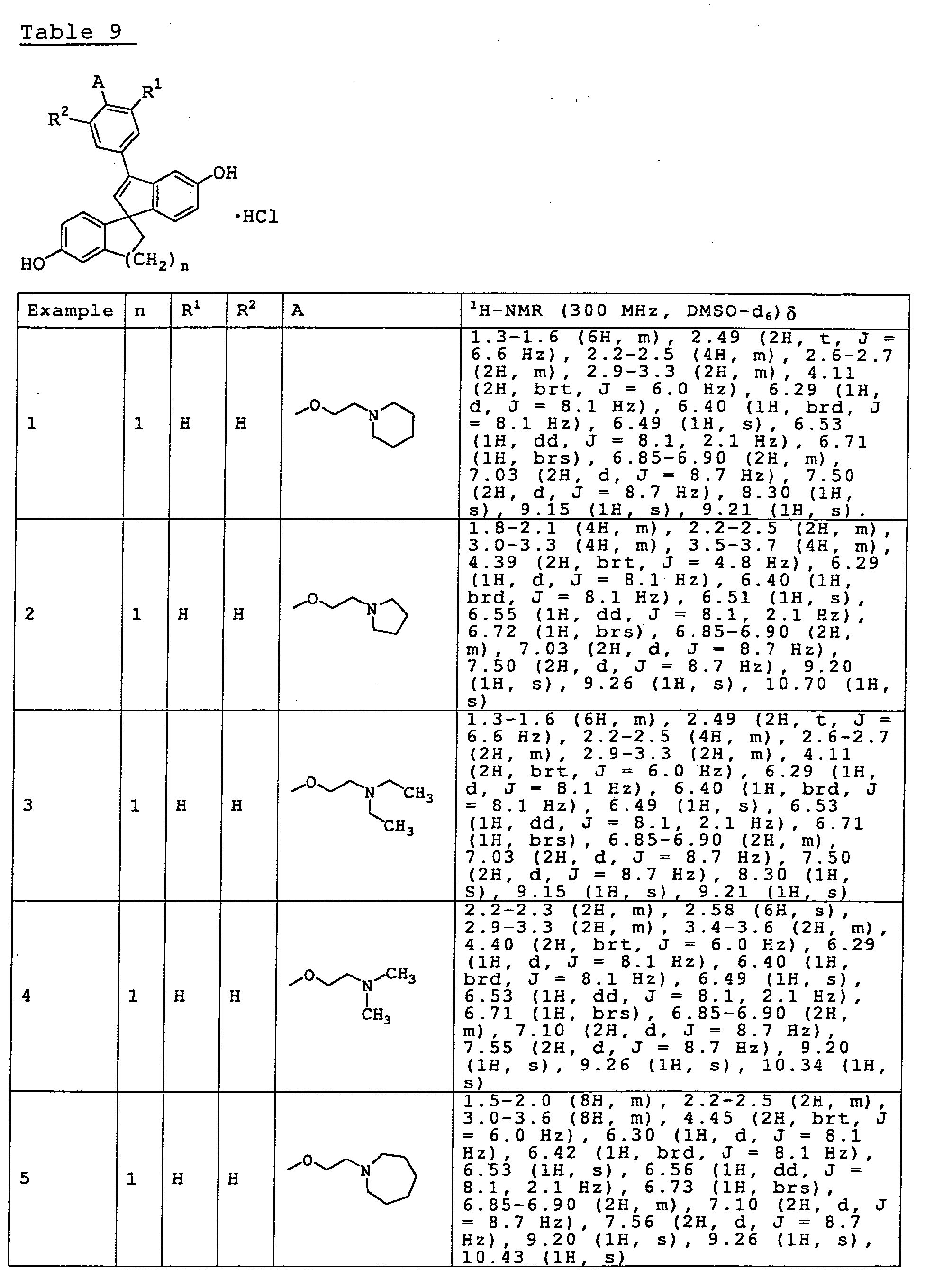

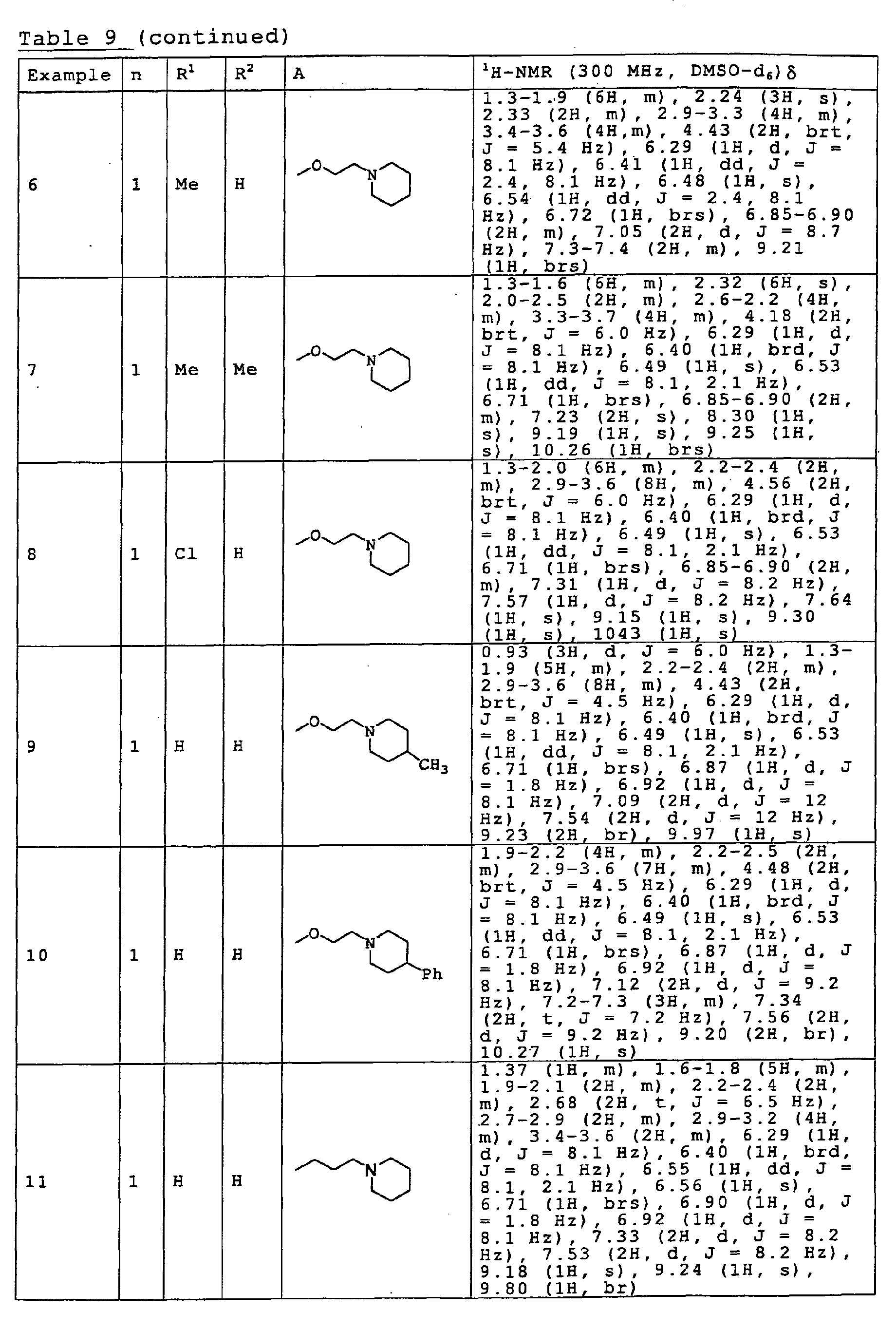

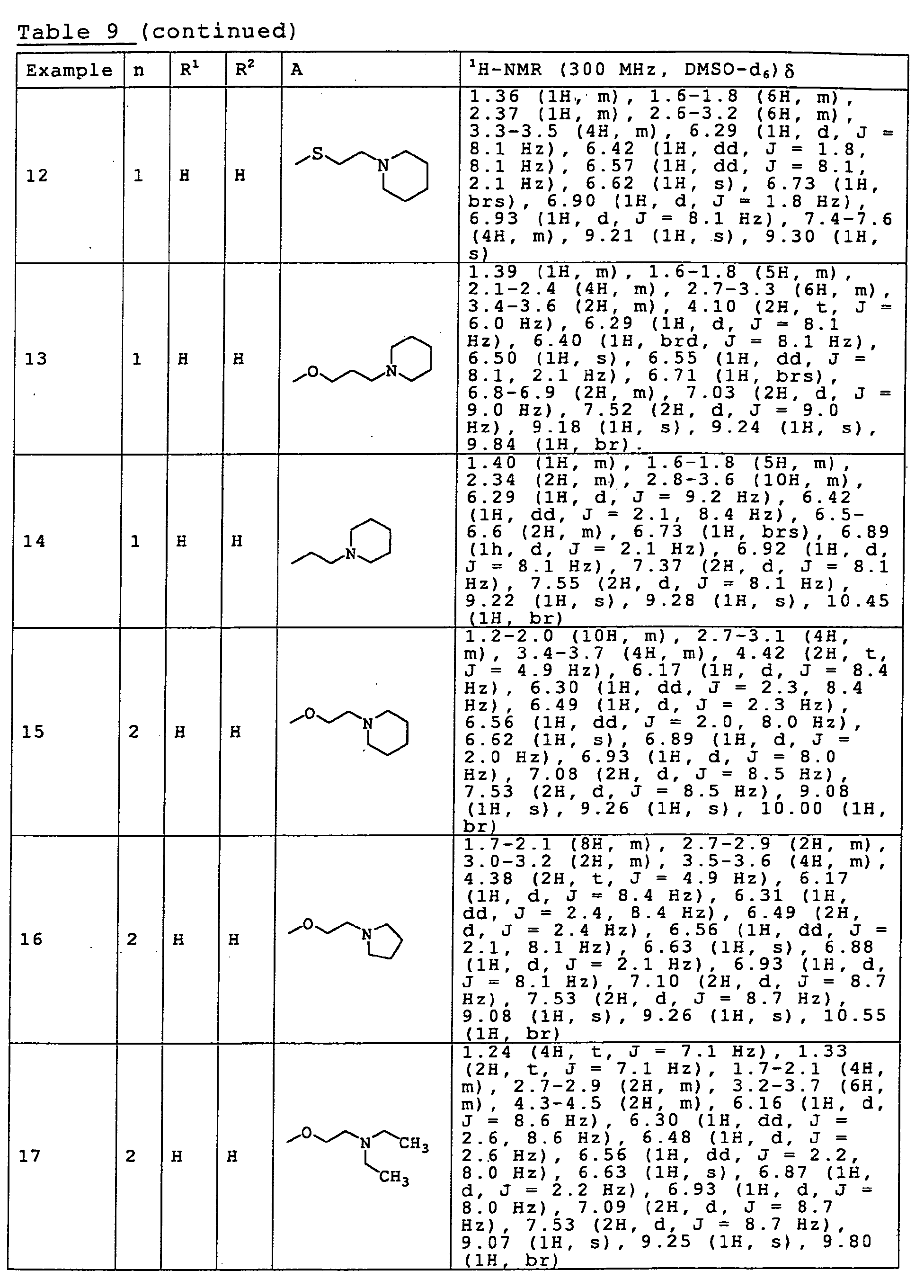

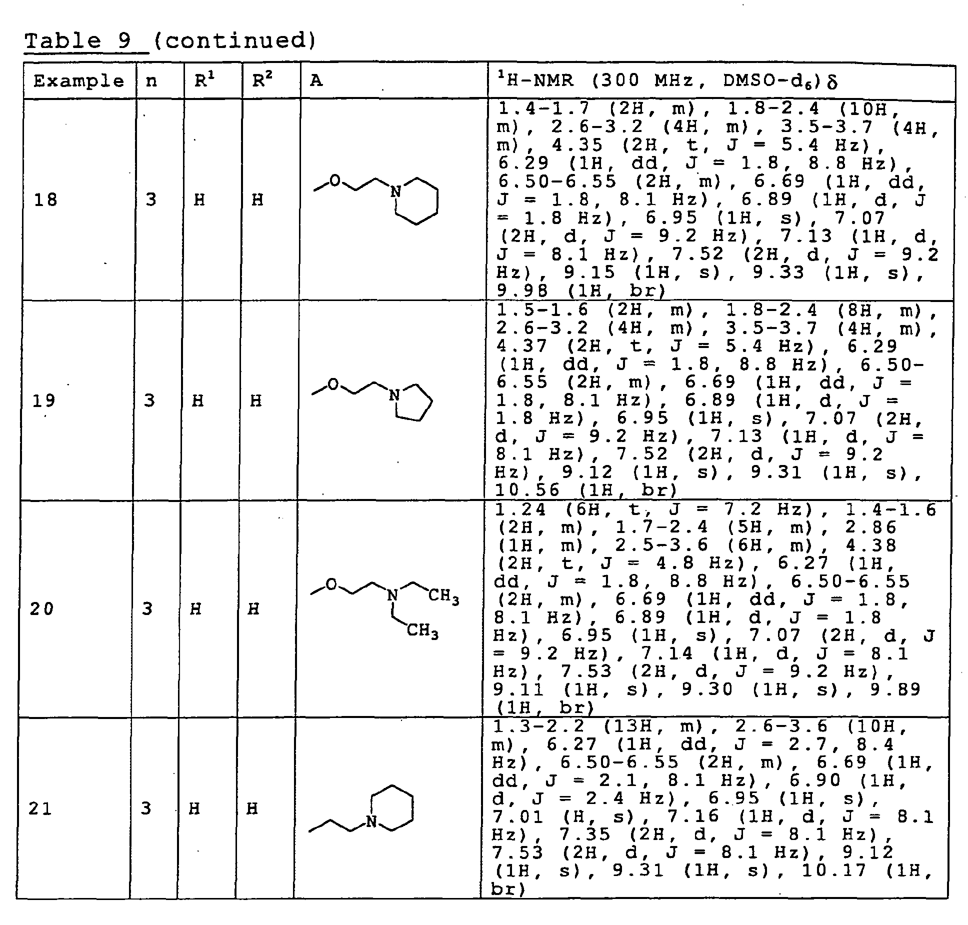

### Example 22

### Production of 3-[4-(4-ethyl-1-piperazinyl)phenyl]spiro[indene-1,1'-indan]-5,5'-diol hydrochloride

The reaction and treatment were performed in the same manner as in Example 1 using 3-[4-(4-ethyl-1-piperazinyl)phenyl]-5,5'-dimethoxyspiro[indene-1,1'-indan] to give the title compound.

The ¹H-NMR data of the title compound are shown in Table 10.

### Examples 23-29

In the same manner as in Example 22 using the corresponding starting compounds, the compounds of Examples 23-29 shown in Table 10 were obtained.

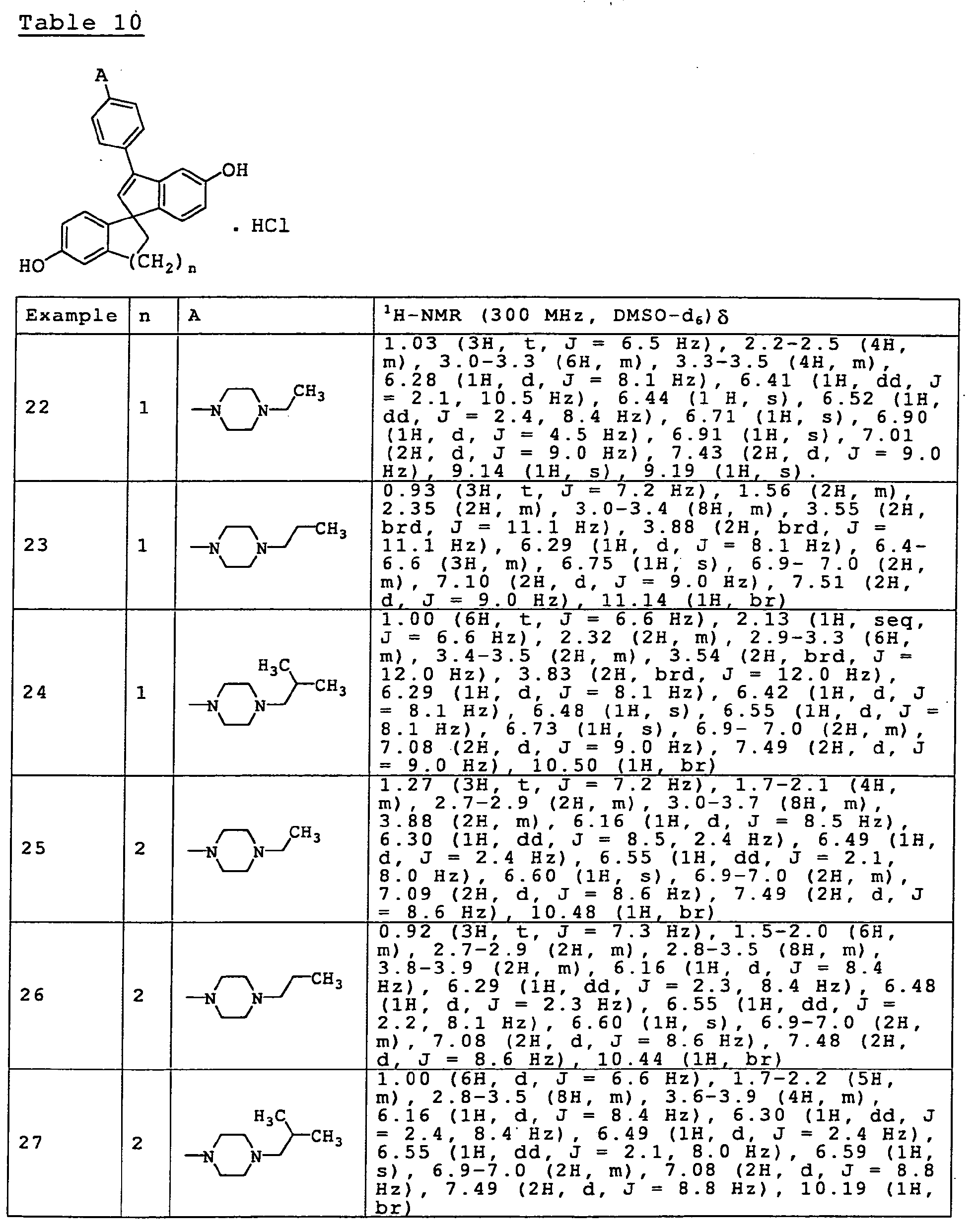

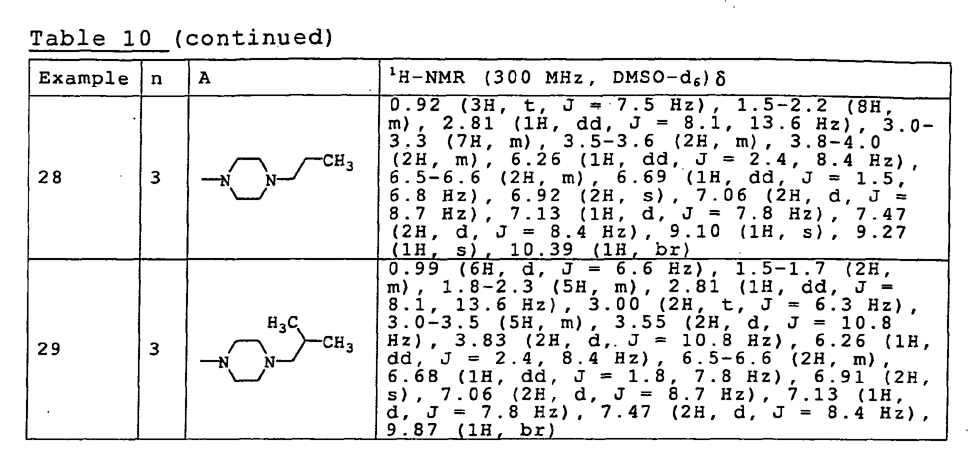

### Example 30

### Production of 3-[4-(1-isobutyl-4-piperidinyl)phenyl]spiro[indene-1,1'-indan]-5,5'-diol hydrochloride

The reaction and treatment were performed in the same manner as in Example 1 using 3-[4-(4-isobutyl-1-piperazinyl)phenyl]-5,5'-dimethoxyspiro[indene-1,1'-indan] to give the title compound.

The ¹H-NMR data of the title compound are shown in Table 11.

### Examples 31-35

In the same manner as in Example 30 using the corresponding starting compounds, the compounds of Examples 31-35 shown in Table 11 were obtained.

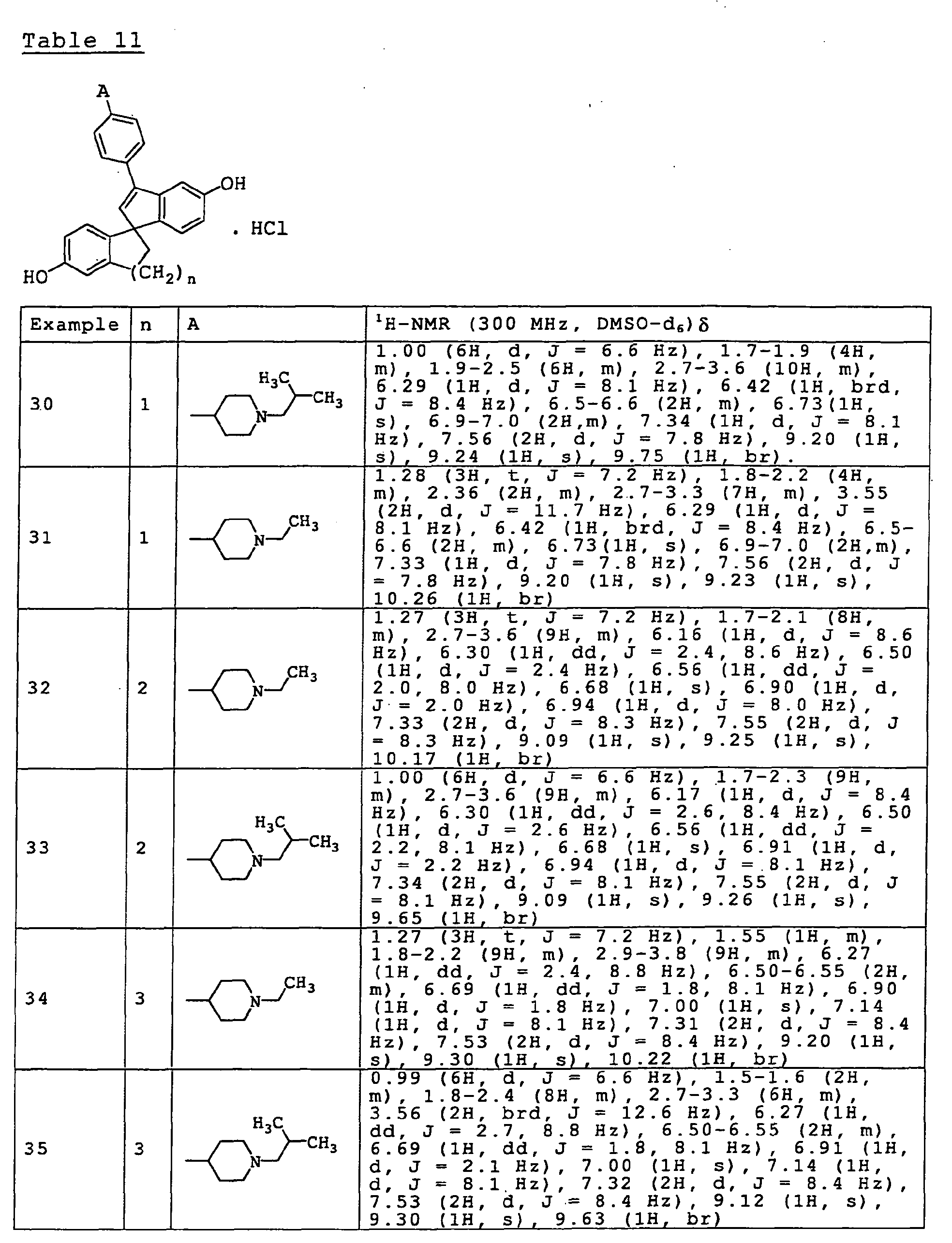

### Example 36

### Production of 3-[4-(2-piperidinoethoxy)phenyl]-1,1'-spirobiindan-5,5'-diol hydrochloride

3-[4-(2-Piperidinoethoxy)phenyl]spiro[indene-1,1'-indan]-5,5'-diol hydrochloride (0.2 g) was dissolved in a mixed solvent of ethanol (3 mL) and acetic acid (0.5 mL), and hydrogenation (1 atm) was performed in the presence of 10% palladium on carbon (0.1 g) at room temperature for 10 hr. The reaction mixture was filtered and the filtered 10% palladium on carbon was washed with ethyl acetate (20 mL). The filtrate and the washing solution were combined and concentrated under reduced pressure. The residue was dissolved in ethyl acetate (20 mL), and washed successively with saturated aqueous sodium hydrogen carbonate solution (10 mL) and saturated brine (10 mL). The organic layer was concentrated under reduced pressure to give the title compound (mixture of two kinds of diastereomers) as an oil.

### Example 36A

### Production of (1R*,3S*)-3-[4-(2-piperidinoethoxy)phenyl]-1,1'-spirobiindan-5,5'-diol hydrochloride

The oil obtained in Example 36 was subjected to basic silica gel column chromatography (ethyl acetate/hexane) to give a free base (0.17 g) of the title compound as an oil. This was dissolved in water-containing methanol, hydrochloric acid was added and the mixture was freeze-dried to give hydrochloride thereof. ¹H-NMR of a free base of the title compound (300 MHz, CD₃OD) δ 1.44 (2H, m), 1.6-1.7 (4H, m), 1.9-2.1 (2H, m), 2.40 (1H, m), 2.5-2.7 (5H, m), 2.77 (2H, t, J = 6.0 Hz), 2.87 (2H, m), 4.11 (2H, t, J = 6.0 Hz), 4.30 (1H, dd, J = 7.2, 11.2 Hz), 6.24 (1H, s), 6.5-6.7 (4H, m), 6.82 (1H, d, J = 8.1 Hz), 6.89 (2H, d, J = 9.0 Hz), 7.16 (2H, d, J = 9.0 Hz).

### Example 36B

### Production of (1R*,3R*)-3-[4-(2-piperidinoethoxy)phenyl]-1,1'-spirobiindan-5,5'-diol hydrochloride

The oil obtained in Example 36 was subjected to basic silica gel column chromatography (ethyl acetate/hexane) to give a free base (0.01 g) of the title compound as an oil. This was dissolved in water-containing methanol, hydrochloric acid was added and the mixture was freeze-dried to give hydrochloride thereof. ¹H-NMR of a free base of the title compound (300 MHz, CD₃OD)δ 1.52 (2H, m), 1.6-2.0 (4H, m), 2.1-2.3 (2H, m), 2.49 (1H, m), 2.56 (1H, dd, J = 8.1, 11.2 Hz), 2.87 (2H, t, J = 6.0 Hz), 3.05 (2H, m), 3.5-3.7 (4H, m), 4.33 (2H, t, J = 6.0 Hz), 4.41 (1H, t, J = 7.2 Hz), 6.38 (1H, s), 6.5-6.7 (4H, m), 6.79 (1H, d, J = 8.1 Hz), 6.85 (2H, d, J = 9.0 Hz), 7.07 (2H, d, J = 9.0 Hz).

### Example 37

### Production of (+)-3-[4-(2-piperidinoethoxy)phenyl]spiro[indene-1,1'-indan]-5,5'-diol hydrochloride

The reaction and treatment were performed in the same manner as in Example 1 to give the title compound from (+)-5,5'-dimethoxy-3-[4-(2-piperidinoethoxy)phenyl]spiro[indene-1,1'-indan].
HPLC analysis; retention time 19.73 min (column; CHIRALCEL OJ, mobile phase;
hexane/ethanol/diethylamine=80/20/1, flow rate; 1.0 mL/min).

| Elemental Analysis (1 hydrochloride 3/4 hydrate): | | | | |
|---|---|---|---|---|
| Calculated | C:71.56; | H:6.71; | N:2.78; | Cl; 7.04: |
| Calculated | C:71.64; | H:6.65; | N:2.83; | Cl; 7.05 |

[α]_{D}²⁴ +51.2° (c 0.41,MeOH)

### Example 38

### Production of (-)-3-[4-(2-piperidinoethoxy)phenyl]spiro[indene-1,1'-indan]-5,5'-diol hydrochloride

The reaction and treatment were performed in the same manner as in Example 1 to give the title compound from (-)-5,5'-dimethoxy-3-[4-(2-piperidinoethoxy)phenyl]spiro[indene-1,1'-indan].
HPLC analysis; retention time 26.66 min (column; CHIRALCEL OJ, mobile phase;
hexane/ethanol/diethylamine=80/20/1, flow rate; 1.0 mL/min).
[α]_{D}²⁴ -51.4° (c 0.52,MeOH)

### Example 39

### Production of 3-[4-((2S)-1-methyl-2-pyrrolidinylmethyloxy)phenyl]spiro[indene-1,1'-indan]-5,5'-diol hydrochloride

The reaction and treatment were performed in the same manner as in Example L and Example 1 using (+)-3-(4-hydroxyphenyl)-5,5'-dimethoxyspiro[indene-1,1'-indan] and (S)-N-methylprolinol as starting materials, whereby the title compound was synthesized.
¹H-NMR (300 MHz, CD₃OD-d₄)δ 1.5-1.8 (3H, m), 1.9-2.1 (2H, m), 2.2-2.6 (3H, m), 2.37 (3H, s), 2.9-3.5 (3H, m), 3.86 (1H, dd, J = 6.0, 9.0 Hz), 4.01 (1H, dd, J = 5.7, 9.0 Hz), 6.29 (1H, d, J = 8.1 Hz), 6.40 (1H, dd, J = 1.8, 8.6 Hz), 6.49 (1H, s), 6.53 (1H, dd, J = 2.1, 8.1 Hz), 6.71 (1H, s), 6.8-6.9 (2H, m), 7.02 (2H, d, J = 8.4 Hz), 7.49 (2H, d, J = 8.4 Hz), 8.30 (1H, s), 9.16 (1H, s), 9.22 (1H, s).

### Example 40

### Production of (+)-3-[4-(4-ethyl-1-piperazinyl)phenyl]spiro[indene-1,1'-indan]-5,5'-diol dihydrochloride

(1) 3-[4-(Trifluoromethanesulfonyloxy)phenyl]-5,5'-dimethoxyspiro[indene-1,1'-indan] (0.5 g) obtained in Example N, potassium phosphate (0.3 g), 1-ethylpiperazine (0.15 mL), palladium acetate (16 mg) and 2-(di-tert-butylphosphino)biphenyl (45 mg) were placed in a flask, and the air was purged with argon. Anhydrous 1,4-dioxane (4 mL) was added thereto and the mixture was heated at 80°C for 20 hr. After allowing to cool, the reaction mixture was filtered and washed with ethyl acetate. The filtrate and the washing solution was combined and the mixture was concentrated under reduced pressure. The residue was subjected to basic silica gel column chromatography (ethyl acetate/hexane) to give the title compound (0.28 g) as an oil.
(2) The above-mentioned oil (0.28 g), diphenylphosphine (0.89 g) and butyl lithium (3 mL) were reacted and treated in the same manner as in Example 1 to give the title compound (0.22 g).

| Elemental Analysis (1.95 hydrochloride 0.70 hydrate): | | | | |
|---|---|---|---|---|
| Calculated | C:67.19; | H:6.64; | N:5.22; | Cl; 12.89: |
| Calculated | C:67.27; | H:6.58; | N:5.13; | Cl; 12.80. |

### Preparation Example 1 Production of tablet

3-[4-(2-Piperidinoethoxy)phenyl]spiro[indene-1,1'-indan]-5,5'-diol (25 g), lactose (60 g), corn starch (16 g), crystalline cellulose (20 g) and hydroxypropyl cellulose (2.3 g) are subjected to mixing, granulation and drying according to conventional methods, and light silicic anhydride (0.6 g) and magnesium stearate (1.1 g) are added. The mixture is punched at 125 mg per tablet to give 1000 tablets.

### Preparation Example 2 Production of capsule

3-[4-(2-Piperidinoethoxy)phenyl]spiro[indene-1,1'-indan]-5,5'-diol (50 g), lactose (117 g), corn starch (25 g), hydroxypropyl cellulose (3.5 g) and purified water (100 g) are subjected to mixing, granulation and drying according to conventional methods, and light silicic anhydride (1.8 g) and magnesium stearate (2.7 g) are added. The granules (200 mg) are filled in capsules to give 1000 capsules.

### Preparation Example 3 Production of powder

3-[4-(2-Piperidinoethoxy)phenyl]spiro[indene-1,1'-indan]-5,5'-diol (200 g), lactose (770 g), hydroxypropyl cellulose (25 g) and light silicic anhydride (5 g) are mixed according to conventional methods and processed to give a powder.

### INDUSTRIAL APPLICABILITY

As explained above, the compound of the formula (I), like the existing SERMs, acts as an estrogen antagonist on the genital organs such as uterus, breast and the like, and also shows an estrogen agonist activity on lipid metabolism, bone, cardiovascular system and brain. Moreover, since the compound of the formula (I) is observed to show a climacteric syndrome-ameliorating effect, it is expected to become a drug for the prophylaxis and/or treatment of osteoporosis and climacteric syndrome or a drug for the prophylaxis and/or treatment of breast cancer, which is more superior to the existing SERMs.

This application is based on a patent application No. 2002-289187 filed in Japan, the contents of which are hereby incorporated by reference.

## Claims

1. A spiro compound represented by the following formula (I): 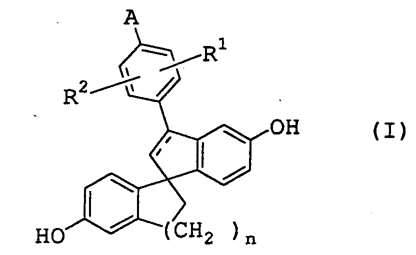 wherein R¹ and R² are the same or different and each is a hydrogen atom, a fluorine atom, a chlorine atom or a C₁₋₆ alkyl group,
n is 1, 2 or 3,
a bond containing a broken line is a single bond or a double bond,
A is -(CH₂)ₚ-N(R³)(R⁴); -X- (CH₂)_{q}-N(R³)(R⁴); a group represented by the following formula (a): 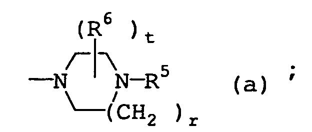 a group represented by the following formula (b) 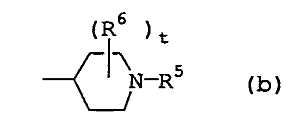 or a group represented by the following formula (c): 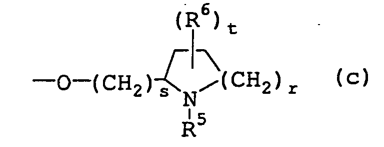 wherein p is 1, 2 or 3,
X is an oxygen atom or a sulfur atom,
q is 2 or 3,
R³ and R⁴ are the same or different and each is a C₁₋₆ alkyl group or a phenyl C₁₋₄ alkyl group, or R³ and R⁴ optionally form, together with the adjacent nitrogen atom, a pyrrolidine ring, a piperidine ring, a homopiperidine ring, a piperazine ring or a morpholine ring, each optionally substituted by one or two groups selected from a C₁₋₆ alkyl, phenyl and benzyl,
R⁵ is a C₁₋₆ alkyl group, a C₃₋₈ cycloalkyl group or a C₃₋₈ cycloalkyl C₁₋₄ alkyl group,
R⁶ is a hydrogen atom or a C₁₋₄ alkyl group, and
r, s and t are each independently one or two, or a pharmaceutically acceptable acid addition salt thereof.

2. The compound of claim 1, wherein A is -X-(CH₂)_{q}-N(R³)(R⁴) (wherein X, R³, R⁴ and q are as defined in claim 1), or a pharmaceutically acceptable acid addition salt thereof.

3. A spiro compound represented by the following formula (I-1a): 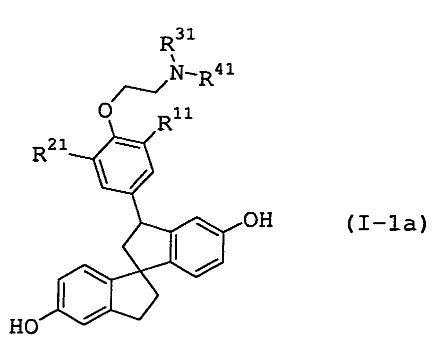 wherein R¹¹ and R²¹ are the same or different and each is a hydrogen atom, a fluorine atom or a chlorine atom,
R³¹ and R⁴¹ form, together with the adjacent nitrogen atom, a pyrrolidine ring, a piperidine ring or a homopiperidine ring, each optionally substituted by one or two methyl groups, or a pharmaceutically acceptable acid addition salt thereof.

4. A spiro compound represented by the following formula (I-2a) : 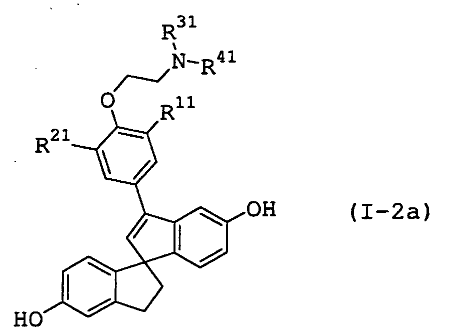 wherein R¹¹ and R²¹ are the same or different and each is a hydrogen atom, a fluorine atom or a chlorine atom,
R³¹ and R⁴¹ form, together with the adjacent nitrogen atom, a pyrrolidine ring, a piperidine ring or a homopiperidine ring, each optionally substituted by one or two methyl groups, or a pharmaceutically acceptable acid addition salt thereof.

5. (1R*,3S*)-3-[4-(2-Piperidinoethoxy)phenyl]-1,1'-spirobiindan-5,5'-diol, or a pharmaceutically acceptable acid addition salt thereof.

6. 3-[4-(2-Piperidinoethoxy)phenyl]spiro[indene-1,1'-indan]-5,5'-diol, or a pharmaceutically acceptable acid addition salt thereof.

7. A compound selected from (+)-3-[4-(2-piperidinoethoxy)phenyl]spiro[indene-1,1'-indan]-5,5'-diol and (-)-3-[4-(2-piperidinoethoxy)phenyl]spiro[indene-1,1'-indan]-5,5'-diol, or a pharmaceutically acceptable acid addition salt thereof.

8. A pharmaceutical composition comprising, as an active ingredient, a compound of any of claims 1 to 7 or a pharmaceutically acceptable acid addition salt thereof.

9. The pharmaceutical composition of claim 8 to be used for the prophylaxis and/or treatment of osteoporosis, climacteric syndrome or breast cancer.

10. Use of a compound of any of claims 1 to 7 or a pharmaceutically acceptable acid addition salt thereof for the prophylaxis or treatment of osteoporosis, climacteric syndrome or breast cancer.

11. A method for the prophylaxis or treatment of osteoporosis, climacteric syndrome or breast cancer, which comprises administering an effective amount of a compound of any of claims 1 to 7 or a pharmaceutically acceptable acid addition salt thereof to a patient with osteoporosis, climacteric syndrome or breast cancer.

12. A commercial package comprising the pharmaceutical composition of claim 8 or 9 and a written matter associated therewith, the written matter stating that the pharmaceutical composition can or should be used for the prophylaxis or treatment of osteoporosis, climacteric syndrome or breast cancer.

13. A spiro compound represented by the following formula (II) : 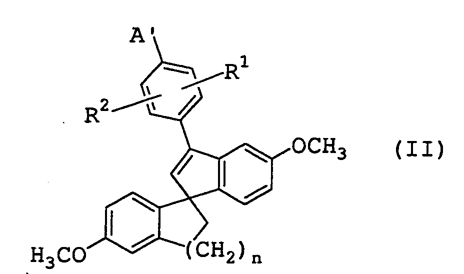 wherein R¹ and R² are the same or different and each is a hydrogen atom, a fluorine atom, a chlorine atom or a C₁₋₆ alkyl group,
n is 1, 2 or 3,
A' is -OH; -OCH₂CH=CH₂; -OSO₂CF₃; -(CH₂)ₚ-N(R³)(R⁴); -X-(CH₂)_{q}-N(R³)(R⁴); a group represented by the following formula (a) 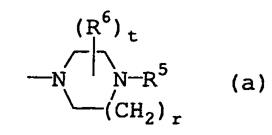 a group represented by the following formula (b) 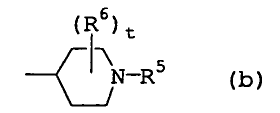 a group represented by the following formula (c) 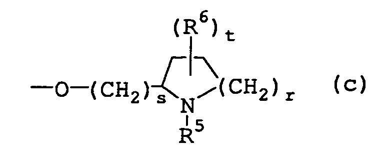 wherein p is 1, 2 or 3,
X is an oxygen atom or a sulfur atom,
q is 2 or 3,
R³ and R⁴ are the same or different and each is a C₁₋₆ alkyl group or a phenyl C₁₋₄ alkyl group, or R³ and R⁴ optionally form, together with the adjacent nitrogen atom, a pyrrolidine ring, a piperidine ring, a homopiperidine ring, a piperazine ring or a morpholine ring, each optionally substituted by one or two groups selected from C₁₋₆ alkyl, phenyl and benzyl,
R⁵ is a C₁₋₆ alkyl group, a C₃₋₈ cycloalkyl group or a C₃₋₈ cycloalkyl C₁₋₄ alkyl group,
R⁶ is a hydrogen atom or a C₁₋₄ alkyl group, and
r, s and t are each independently one or two.

14. A spiro compound represented by the following formula (III): 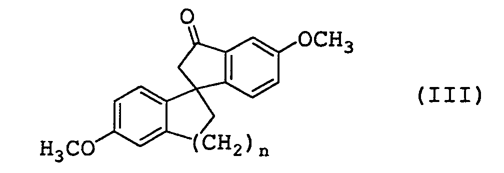 wherein n is 1, 2 or 3.
